# EUROPEAN PATENT APPLICATION

(11) **EP 4 528 017 A1**
(43) Date of publication of application: **26.03.2025**
(21) Application number: 23827443.5
(22) Date of filing: 15.06.2023
(51) Int. Cl.: D06F 58/18, D06F 58/22, D06F 58/36, D06F 34/20, D06F 33/63, A61L 2/10, D06F 58/20, D06F 103/42, D06F 105/32, D06F 105/58

(54) **CLOTHING TREATMENT APPARATUS**

(30) Priority: 20.06.2022 KR 20220075190; 14.03.2023 KR 20230033148
(71) Applicant: LG Electronics Inc., Yeongdeungpo-gu Seoul 07336 (KR)
(72) Inventor: LEE, Heebeom, Seoul 08592 (KR); KIM, Jaehyung, Seoul 08592 (KR); YOON, Taejun, Seoul 08592 (KR)
(74) Representative: Vossius & Partner Patentanwälte Rechtsanwälte mbB
(86) International application number: PCT/KR2023/008261
(87) International publication number: WO 2023/249324

(57) **Abstract**

**ABSTRACT:** The present disclosure relates to a clothing treatment apparatus comprising a light emitting part which is arranged to be spaced apart from a filter part and is provided to emit light toward the filter part, and a sensor part provided to detect the light which is emitted from the light emitting part and passes through the filter part, and further comprising a control part provided to detect one or more of whether the filter part is attached or detached or a degree of contamination of the filter part according to the amount of light emitted from the light emitting part detected by the sensor part.

## Description

### TECHNICAL FIELD

The present disclosure relates to a clothing treatment apparatus. More particularly, the present disclosure relates to a clothing treatment apparatus capable of determining whether a filter is attached or detached through an illuminance sensor.

### BACKGROUND ART

In general, clothing treatment apparatuses conceptually include a washer that wets clothes in water to make it wet and then removes foreign substances through chemical action of detergent and physical action, such as drum rotation, and a dryer that dries the wet clothes using hot air and steam.

However, recently, a clothing care system that keeps dry clothes comfortable and clean without wetting thereof has appeared. This clothing care system may perform a refreshing cycle of removing odors from the clothes and drying or sterilizing the clothes by supplying steam or hot air to the clothes in a state in which the clothes are held.

This clothing care system may selectively add fragrance to the clothes, and has recently taken up an important role in clothing treatment apparatuses along with the washer and dryer.

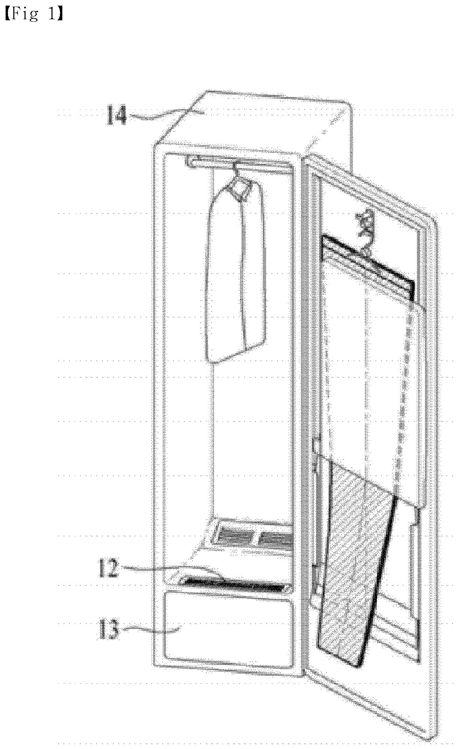

FIG. 1 illustrates a conventional clothing treatment apparatus provided with a steam supply.

Referring to Korean Patent Laid-open Publication No. 10-2020-0057545, a conventional clothing treatment apparatus includes a cabinet 1 forming the appearance of the apparatus, and an inner case 2 provided in the cabinet 1 to accommodate clothes .

The clothing treatment apparatus may have a machine compartment below the inner case 2. The machine compartment may have a circulation duct 13 that circulates air of the inner case 2, a heat exchanger 15 provided in the circulation duct 13 to exchange heat with the air, and a compressor 14 configured to supply a high-temperature refrigerant to the heat exchanger 15. When the compressor 14 is operated, hot air may be supplied to the inner case 2 to raise the temperature in the inner case 2 to dry or sterilize the clothes.

In addition, the clothing treatment apparatus may include a filter on the upper portion of the machine compartment to prevent dust from entering the inside of the circulation duct 13.

However, there was a problem in that the user would operate the clothing treatment apparatus while not installing the filter when using the clothing treatment apparatus, and if the filter was not installed, there is a high possibility that dust of large particles will enter the inside of the circulation duct 13, which may cause sanitation problems.

Accordingly, a clothing treatment apparatus that determines whether a filter is attached or detached using a reed switch installed on the filter has appeared.

However, the reed switch is greatly affected by shock and vibration due to the mechanical characteristics thereof, causes separation of a contact part of the reed switch and reduces stability of the corresponding part, and is thus limited in accuracy.

In addition, there was a problem in that a mechanical reed switch wore out over time, and the lifespan thereof was thus limited.

Further, referring to Korean Patent Laid-open Publication No. 10-1995-0008839, a clothing treatment apparatus that detects clogging of a filter using light has appeared. However, since the conventional clothing treatment apparatus does not directly emit light to the filter, there was a problem in that it was not possible to determine whether the filter is not installed.

As a result, there was a problem in that the clothing treatment apparatus was not capable of accurately determining whether the filter was installed, and thus the clothing treatment apparatus was operated even when the filter was not installed.

### DISCLOSURE

### TECHNICAL TASK

One technical task of the present disclosure is to provide a clothing treatment apparatus for detecting whether a filter is attached or detached using an illuminance sensor.

Another technical task of the present disclosure is to provide a clothing treatment apparatus that may determine whether a filter is attached or detached from an increase in the illuminance of light detected by a sensor unit when the filter is not installed.

Another technical task of the present disclosure is to provide a clothing treatment apparatus that may determine whether a filter is attached or detached using an illuminance sensor that is less affected by shock and vibration and has no limit to the life of a light source as long as power is supplied.

Another technical task of the present disclosure is to provide a clothing treatment apparatus that may indirectly and quickly determine whether a filter is attached or detached by detecting a change in the illuminance of light without direct contact of a sensor with the filter.

Another technical task of the present disclosure is to provide a clothing treatment apparatus that may determine whether a filter is attached or detached by detecting a change in the illuminance of light by a sensor unit as long as light emitted from a light emitting unit reaches the sensor unit regardless of where the light emitting unit and the sensor unit are provided.

Another technical task of the present disclosure is to provide a clothing treatment apparatus that may determine a contamination level of a filter using an illuminance sensor.

### TECHNICAL SOLUTIONS

In order to solve the above technical tasks, the present disclosure provides a clothing treatment apparatus including a light emitting unit disposed to be spaced apart from a filter unit and provided to emit light toward the filter unit, and a sensor unit provided to detect the light emitted from the light emitting unit and having passed through the filter unit, and further including a controller provided to detect at least one of whether the filter unit is attached or detached or a contamination level of the filter unit depending on an amount of the light emitted from the light emitting unit, detected by the sensor unit.

The filter unit may be disposed between the light emitting unit and the sensor unit.

One of the light emitting unit and the sensor unit may be disposed above the filter unit, and a remaining one is disposed below the filter unit.

The light emitting unit may be disposed in an inflow body, and the sensor unit may be disposed in an inner case or a door.

The sensor unit may be disposed closer to the filter unit than a hanger unit.

The clothing treatment apparatus may further include a reflection unit disposed above the light emitting unit and the sensor unit and provided to guide the light emitted from the light emitting unit to the sensor unit.

The filter unit may include a body filter mounted on the inflow body and a filter cover disposed above the body filter, and the reflection unit may be disposed between the filter cover and the body filter.

One of the light emitting unit and the sensor unit may be disposed below the filter unit, and a remaining one of the light emitting unit and the sensor unit may be disposed on an upper surface of the inner case.

The clothing treatment apparatus may further include a hanger unit provided in an upper portion of the inner case to hold the clothes, and one of the light emitting unit and the sensor unit may be disposed below the filter unit and the hanger unit.

The light emitting unit and the sensor unit may be disposed in the inflow body to prevent exposure thereof to the inner case.

The filter unit may include a body filter mounted on the inflow body and a filter cover disposed above the body filter, and the light emitting unit and the sensor unit may be disposed below the filter cover.

The light emitting unit and the sensor unit may be disposed below the body filter, and the clothing treatment apparatus may further include a reflection unit provided to guide the light emitted from the light emitting unit to the sensor unit through the body filter.

The present disclosure provides a clothing treatment apparatus including a light emitting unit disposed to be spaced apart from a filter unit and provided to emit light toward the filter unit, a sensor unit provided to detect the light emitted from the light emitting unit and having passed through the filter unit, and a controller provided to detect whether the filter unit is attached or detached depending on an amount of the light emitted from the light emitting unit, detected by the sensor unit, wherein the sensor unit detects a first value when the filter unit is installed on the inflow body, and the controller determines that the filter unit is not installed on the inflow body when the sensor unit detects a value greater than the first value.

The machine compartment may further include a steam generator configured to generate steam to be supplied to an inner case, and a compressor configured to compress a refrigerant provided to exchange heat with air supplied to the inner case, and the controller may be provided to shut down operation of at least one of the steam generator or the compressor upon determining that the filter unit is not installed.

The sensor unit may detect a second value when the filter unit is not contaminated, the second value may be smaller than the first value, and the controller may determine that the filter unit is contaminated when a value smaller than the second value is detected.

The clothing treatment apparatus may further include a display provided on one of a cabinet and a door to display a state of the filter unit, and the controller may be provided to display contamination information of the filter unit on the display upon determining that the filter unit is contaminated.

The present disclosure provides a clothing treatment apparatus including a light emitting unit disposed to be spaced apart from a filter unit and provided to emit light toward the filter unit, wherein the light emitting unit is provided to sterilize a body filter through the light.

The light emitting unit may be provided to emit visible light having a wavelength of 405 nm.

A chemical substance configured to emit a sterilizing substance upon coming into contact with the visible light may be applied to the filter unit.

The light emitting unit may be provided to emit light in an ultraviolet spectrum.

### ADVANTAGEOUS EFFECTS

The present disclosure has the effect of preventing erroneous use of a clothing treatment apparatus and reducing sanitation problems by detecting whether a filter is attached or detached using an illuminance sensor.

The present disclosure has the effect of being capable of determining whether a filter is attached or detached using an illuminance sensor that is less affected by shock and vibration and has no limit to the life of a light source as long as power is supplied.

The present disclosure has the effect of being capable of indirectly and quickly determining whether a filter is attached or detached by detecting a change in the illuminance of light without direct contact of a sensor with the filter.

The present disclosure has the effect of being capable of determining whether a filter is attached or detached by detecting a change in the illuminance of light by a sensor unit as long as light emitted from a light emitting unit reaches the sensor unit regardless of where the light emitting unit and the sensor unit are provided.

The present disclosure has the effect of being capable of determining the contamination level of a filter using an illuminance sensor.

### DESCRIPTION OF DRAWINGS

FIG. 1 illustrates a conventional clothing treatment apparatus.
FIG. 2 illustrates the appearance of a clothing treatment apparatus of the present disclosure.
FIG. 3 illustrates the structure of an upper portion of an inner case.
FIG. 4 illustrates the structure of a machine compartment of the clothing treatment apparatus of the present disclosure.
FIG. 5 illustrates the structure of a base of the machine compartment of the clothing treatment apparatus of the present disclosure.
FIG. 6 illustrates the structure of a circulation duct of the clothing treatment apparatus of the present disclosure.
FIG. 7 illustrates the shape of the circulation duct of the clothing treatment apparatus of the present disclosure.
FIG. 8 is a cross-sectional view of the circulation duct.
FIG. 9 illustrates the structure of a controller installation unit provided on the base of the clothing treatment apparatus of the present disclosure.
FIG. 10 illustrates the structure of an air discharge unit (323) of the clothing treatment apparatus of the present disclosure.
FIG. 11 illustrates the structure of a base cover of the clothing treatment apparatus of the present disclosure.
FIG. 12 illustrates the structure of an external air duct.
FIG. 13 illustrates the flow of air flowing through the circulation duct.
FIG. 14 illustrates the installation structure of a steam supply.
FIG. 15 illustrates the detailed structure of the steam supply.
FIG. 16 illustrates a filter unit of the clothing treatment apparatus of the present disclosure.
FIG. 17 illustrates position and area division of the clothing treatment apparatus of the present disclosure.
FIG. 18 illustrates one embodiment of positions of a light emitting unit and a sensor unit of the clothing treatment apparatus of the present disclosure.
FIG. 19 illustrates another embodiment of the positions of the light emitting unit and the sensor unit of the clothing treatment apparatus of the present disclosure.
FIG. 20 illustrates another embodiment of the positions of the light emitting unit and the sensor unit of the clothing treatment apparatus of the present disclosure.
FIG. 21 illustrates another embodiment of the positions of the light emitting unit and the sensor unit of the clothing treatment apparatus of the present disclosure.
FIG. 22 illustrates another embodiment of the positions of the light emitting unit and the sensor unit of the clothing treatment apparatus of the present disclosure.
FIG. 23 illustrates another embodiment of the positions of the light emitting unit and the sensor unit of the clothing treatment apparatus of the present disclosure.
FIG. 24 illustrates another embodiment of the positions of the light emitting unit and the sensor unit of the clothing treatment apparatus of the present disclosure.
FIG. 25 illustrates the structure of a first reflector.
FIG. 26 illustrates one embodiment of a reflection unit of the clothing treatment apparatus of the present disclosure.
FIG. 27 illustrates another embodiment of the reflection unit of the clothing treatment apparatus of the present disclosure.
FIG. 28 illustrates the structure of a filter cover of the clothing treatment apparatus of the present disclosure.
FIG. 29 illustrates one embodiment of the positions of the light emitting unit and the sensor unit for sterilizing the filter unit of the clothing treatment apparatus of the present disclosure.
FIG. 30 illustrates a control method of sterilizing the filter unit of the clothing treatment apparatus of the present disclosure.
FIG. 31 illustrates another control method of sterilizing the filter unit of the clothing treatment apparatus of the present disclosure.

### BEST MODE FOR DISCLOSURE

Hereinafter, embodiments of the disclosure will be described in detail with reference to the accompanying drawings. In the present disclosure, the same or similar components are indicated by the same or similar reference numbers even in different embodiments, and the description thereof is replaced with the first description. In the following description of the present disclosure, singular expressions encompass plural expressions unless the context clearly indicates otherwise. In addition, in describing the embodiments of the present disclosure, if it is determined that the detailed descriptions of related known technologies may obscure the gist of the embodiments of the present disclosure, the detailed descriptions will be omitted. Further, it should be understood that the accompanying drawings are provided only for ease of understanding the embodiments of the present disclosure, and the technical idea of the present disclosure is not limited by the accompanying drawings.

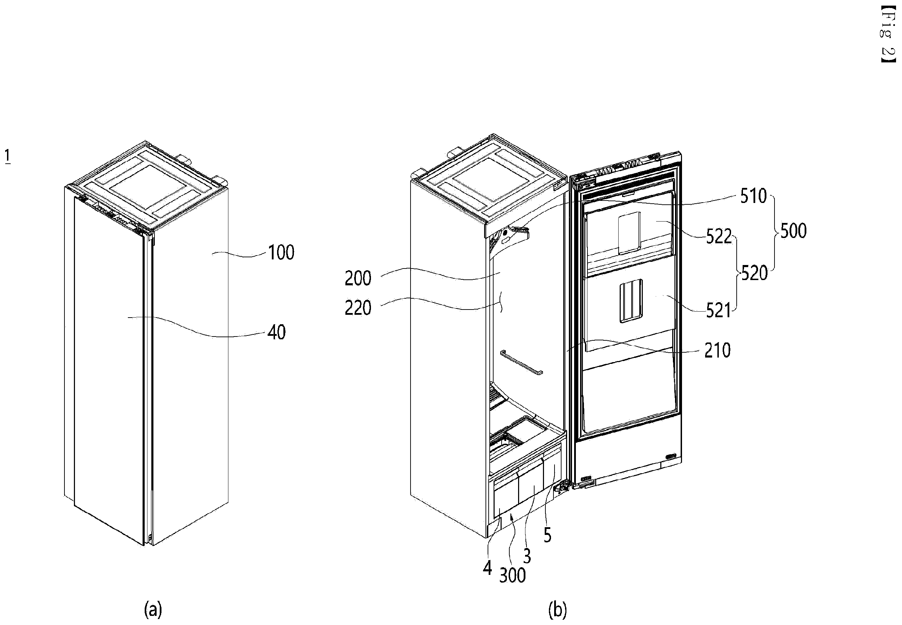

FIG. 2 illustrates the appearance of a clothing treatment apparatus 1 of the present disclosure.

Referring to FIG. 2(a), the clothing treatment apparatus of the present disclosure may include a cabinet 100 forming the appearance of the clothing treatment apparatus, and a door 40 rotatably coupled to the cabinet 100.

The door 40 may include a main body 410 forming the front surface of the cabinet 100, and an installation body 420 extending from one side of the main body 410 so that a display configured to display information of the clothing treatment apparatus may be installed thereon.

The installation body 420 may be provided to form a step 43 toward the rear of the cabinet 100 from the main body 410.

Further, at least a portion of the installation body 20 may be disposed to overlap the main body 410 in the forward and rearward directions at the rear of the main body 410. Thereby, the step 43 may function as a handle.

The installation body 420 may be formed of a different material or in a different color from the main body 410. In addition, the installation body 420 may be formed of a translucent material that transmits light emitted from the display.

Referring to FIG. 2(b), an inner case 200 having an accommodation space 220 to accommodate clothes may be provided within the cabinet 100. The inner case 200 may be provided with an opening 210 formed at the front portion thereof so that the clothes enter and exit the inner case 200 therethrough, and the opening 210 may be shielded by the door 40.

The inner case 200 may be formed of a plastic resin-based material, and may be formed of a reinforced plastic resin-based material that is not deformed even by air of a higher temperature than room temperature, heated air (hereinafter, hot air), steam, or moisture.

The inner case 200 may be provided such that the length thereof is longer than the width thereof. Accordingly, the clothes may be accommodated in the accommodation space 220 without being folded and wrinkled.

The clothing treatment apparatus 1 of the present disclosure may include a holding unit 500 that may hold the clothes in the accommodation space 220 of the inner case 200.

The holding unit 500 may include a hanger unit 510 provided on the upper surface of the inner case 200 to hold the clothes.

When the clothes are held on the hanger unit 510, the clothes may be disposed in a state of floating in the air within the accommodation space 220.

Further, the holding unit 500 may further include a pressing unit 520 coupled to the inner surface of the door 40 to fix the clothes.

The hanger unit 510 may be provided in a bar shape arranged in the width direction of the inner case 200 to support hangers on which clothes are held. Further, as illustrated, the hanger unit 510 may be provided in a hanger shape to allow clothes to be directly held thereon.

The clothing treatment apparatus of the present disclosure may further include a vibration unit that vibrates the hanger unit 510 to remove foreign substances, such as fine dust, attached to the clothes.

The holding unit 500 may include the pressing unit 520 provided on the door 40 to press and fix the clothes. The pressing unit 520 may include a support 522 fixed to the inner surface of the door 40 to one surface of the clothes, and a presser 521 configured to press the clothes supported by the support 522.

The presser 521 may be provided to move toward the support 522 or away from the support 522. For example, the presser 521 may be rotatably provided on the inner surface of the support 522 or the door 40.

As such, the presser 521 and the support 522 may press both sides of the clothes to remove wrinkles from the clothes and create intended creases.

The clothing treatment apparatus of the present disclosure may have a machine compartment 300 equipped with various devices that may supply at least one of hot air or steam to the accommodation space 220 or purify or dehumidify external air of the cabinet 100.

The machine compartment 300 may be disposed to be separated or partitioned from the inner case 200, but may be provided to communicate with the inner case 200.

The machine compartment 300 may be disposed below the inner case 200. Accordingly, when hot air and steam with low specific gravity are supplied to the inner case 200, the hot air and steam may be naturally supplied to the clothes.

The machine compartment 300 may include a heat supply 340 that may supply hot air to the inside of the inner case 200. The heat supply 340 may be provided as a heat pump system, or may be provided as a heater that directly heats air with electric energy.

If the heat supply 340 is provided as a heat pump system, it may be provided to dehumidify and heat air discharged from the inner case 200 again and supply the air to the inner case 200. The detailed structure of the heat supply 340 will be described later.

The machine compartment 300 may include a steam supply 800 that may supply steam to the inside of the inner case 200. The steam supply 800 may be provided to directly supply steam to the inside of the inner case 200. The detailed structure of the steam supply 800 will be described later.

For this purpose, the inner case 200 may have a plurality of through holes 230 formed through one surface of the inner case 200 to communicate with the machine compartment 300.

Through the through holes 230, air inside the accommodation space 220 may be supplied to the machine compartment 300, and at least one of hot air or steam generated in the machine compartment 300 may be supplied to the accommodation space 200.

The through holes 230 may include an inflow hole 231 formed through the lower surface of the inner case 200 so that the air inside the inner case 200 is suctioned or discharged to the machine compartment 300 therethrough, and a discharge hole 232 formed through the lower surface of the inner case 200 so that the hot air generated in the machine compartment 300 is discharged therethrough.

The discharge hole 232 may be disposed in a portion of the lower surface of the inner case 200 close to the rear surface thereof. For example, the discharge hole 232 may be disposed to be inclined from the ground between the lower surface and the rear surface of the inner case 200 to face the hanger unit 510.

In addition, the inflow hole 231 may be disposed in a portion of the lower surface of the inner case 200 close to the front thereof. Accordingly, the inflow hole 231 may be disposed to be spaced apart from the discharge hole 232.

The through holes 230 may include a steam hole 233 through which steam generated in the steam supply 800 is supplied. The steam hole 233 may be disposed on one side of the discharge hole 232.

Further, a water supply tank 3 capable of supplying water to the steam supply 800 and a drain tank 4 that collects condensed water from the heat supply 340 may be provided in a front portion of the machine compartment 300.

The water supply tank 3 and the drain tank 4 may be detachably provided in the front portion of the machine compartment 300. Accordingly, the clothing treatment apparatus 1 of the present disclosure may be freely installed without being restricted by a water supply source or a drain source.

Meanwhile, a drawer 5 that is withdrawn from and inserted into the front portion of the machine chamber 300 and has a separate storage space may be further provided in the front portion of the machine chamber 300. A steam generator or an iron may be stored in the drawer 5.

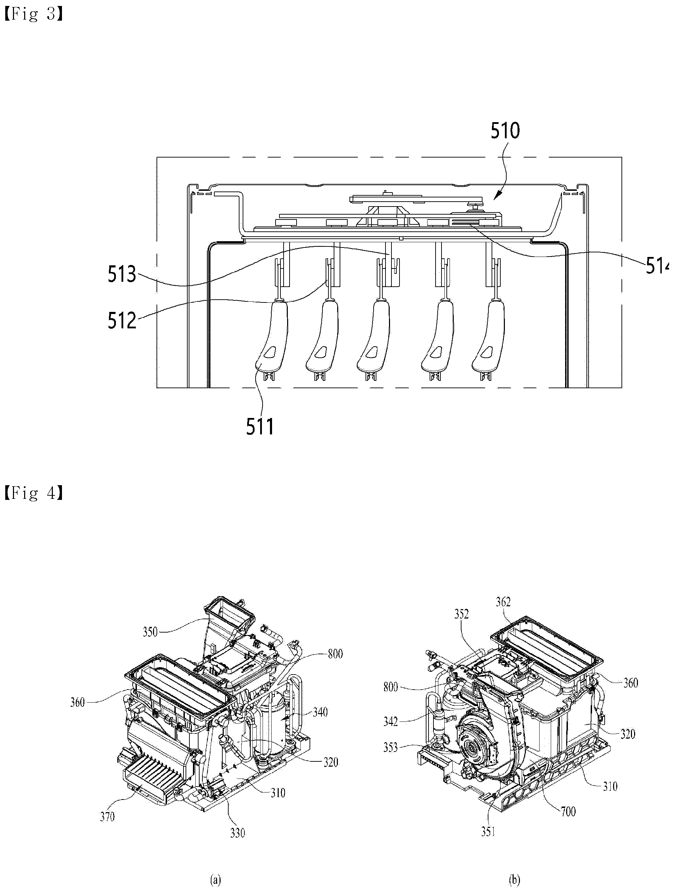

FIG. 3 illustrates the structure of the upper portion of the inner case.

The hanger unit 510 of the clothing treatment apparatus of the present disclosure may include power transmission units 513 disposed in the upper portion of the inner case 200 and provided to shake the hangers 511.

A hook 512 on which the hanger 511 is placed or held may be provided at the lower portion of the power transmission unit 513.

Accordingly, when the power transmission units 513 move, the hooks 512 move, and the hangers 511 held on the hooks 512 shake, thereby exhibiting the effect of shaking off the clothes.

The power transmission units 513 may be provided in plural, and the hooks 512 coupled to the power transmission units 513 may be provided in plural. Accordingly, a large number of pieces of clothes corresponding to the number of the power transmission units 513 may be held in the inner case 200 to be refreshed.

FIG. 3 illustrates that the hanger unit 510 and the power transmission units 513 are arranged in the width direction of the inner case 200.

The hanger unit 510 and the power transmission units 513 may be arranged in a direction in which the clothes are arranged in the accommodation space. For example, if the clothes are arranged in the forward and rearward directions in the inner case 200, the hanger unit 510 and the power transmission units 513 may be arranged in the forward and rearward directions of the inner case 200.

Hereinafter, the hanger unit 510, the plurality of power transmission units 513, and the plurality of hooks 512 will be described as being arranged in the width direction of the inner case 200, but this is only an example, and the hanger unit 510, the plurality of power transmission units 513, and the plurality of hooks 512 may be arranged in a direction designed so that a plurality of pieces of clothes are held or arranged in the accommodation space.

The hanger unit 510 may further include a driver 514 that provides power to move the power transmission units 513.

The driver 514 may be provided to be exposed to the inside of the inner case 200 as long as the driver 514 is capable of transmitting power to the power transmission units 513. However, since the driver 514 is provided to be operated by receiving electric energy, exposure of the driver 514 to steam or hot air is preferably prevented.

Therefore, the driver 514 may be disposed between the upper surface of the inner case 200 and the cabinet 100 so that exposure of the driver 514 to the accommodation space 210 may be prevented.

The power transmission units 513 may penetrate the upper surface of the inner case 200 to receive power from the driver 514. The power transmission units 513 penetrate the upper surface of the inner case 200 and extend downward so that the lower ends of the power transmission units 513 may be exposed to the accommodation space 210.

The power transmission units 513 may be provided in a rod shape, a tube shape, a plate shape, or the like in which the length thereof is longer than the thickness thereof.

FIG. 4 illustrates the structure of the machine compartment of the clothing treatment apparatus of the present disclosure.

FIG. 4(a) is a front view of the machine compartment 300, and FIG. 4(b) is a rear view of the machine compartment 300.

Components configured to supply hot air to a clothing treatment space, circulate air inside the clothing treatment space, supply steam to the clothing treatment space, or purify air outside the cabinet may be disposed within the machine compartment 300.

The machine compartment 300 may include a base unit 310 configured such that various devices are supported thereby or installed thereon. The base unit 310 may provide an area where the various devices are installed.

A circulation duct 320 through which air introduced from the inner case 200 or the outside of the cabinet 100 moves may be installed on the base unit 310.

The circulation duct 320 may be provided in a case shape with an open upper surface, and some of the components of the heat supply 340 may be installed in the circulation duct 320.

If the heat supply 340 is provided as a heat pump system, it may include heat exchangers 341 and 343, which will be described later, and a compressor 342 that supplies a high-temperature and high-pressure refrigerant to the heat exchangers in the circulation duct 320.

The heat exchangers 341 and 343 may be accommodated in the circulation duct 320 to cool and dehumidify the air flowing through the circulation duct 320 or heat the air to generate hot air.

If the circulation duct 320 is provided to draw in air from the outside of the cabinet 100, an external air duct 370 configured to draw in external air may be installed in front of the circulation duct 320.

The circulation duct 320 may be provided to communicate with the external air duct 370 to selectively draw in external air.

The water supply tank and the drain tank may be detachably connected to the front of the circulation duct 320. The water supply tank 3 and the drain tank 4 may be disposed to be placed above the external air duct 370.

The circulation duct 320 may be provided to be coupled to the base unit 310, or may be provided integrally with the base unit 310. For example, the base unit 310 and the circulation duct 320 may be manufactured by injection molding.

The machine compartment 300 may include a base cover 360 provided to allow the circulation duct 320 to communicate with the inflow hole 231.

The base cover 360 may be provided to be coupled to the upper portion of the circulation duct 320 so as to guide air suctioned through the inflow hole 231 into the inside of the circulation duct 320.

The base cover 360 may shield the upper surface of the circulation duct 320 to prevent discharge of air inside the circulation duct 320 to the outside. The lower portion of the base cover 360 and the upper surface of the circulation duct 20 may form one surface of the flow path of the circulation duct 320.

The base cover 360 may include an inflow part 362 that connects the inflow hole 231 and the circulation duct 320. The inflow part 362 may be provided in a duct shape and serve as an intake duct that delivers air inside the inner case 200 to the circulation duct 320.

The steam supply 800 connected to the water supply tank 3 to receive water, generate steam, and supply the generated steam to the inner case 200 may be installed in the machine compartment 300. The steam supply 800 may be disposed to be placed on the base cover 360.

The steam supply 800 may be disposed at the rear of the inflow part 362.

The machine compartment 300 may include a fan installation unit 350 provided to allow the circulation duct 320 to communicate with the inner case 200. The fan installation unit 350 may include a blower fan 353 that provides power to move air inside the circulation duct 320 in one direction, and a fan housing 351 that accommodates the blower fan 353 and is coupled to or extends toward the circulation duct 320.

The fan installation unit 350 may further include a discharge duct 352 provided to allow the circulation duct 320 to communicate with the discharge hole 232.

The discharge duct 352 may be provided to extend from lower portion of the fan housing 351 toward the discharge hole 232 to have a cross-sectional shape and a cross-sectional area corresponding to the discharge hole 232.

As a result, the air inside the inner case 200 may be introduced through the base cover 360, pass through the circulation duct 320, and then be supplied back to the inside of the inner case 200 through the fan installation unit 350.

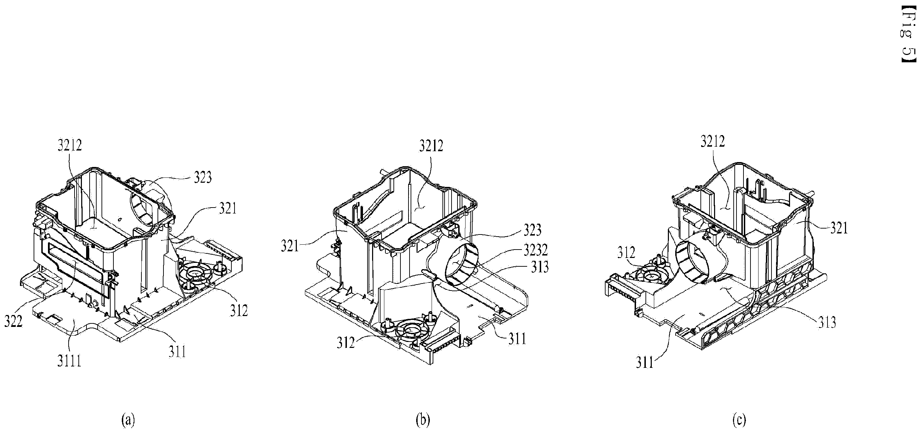

FIG. 5 illustrates the structure of the base of the machine compartment of the clothing treatment apparatus of the present disclosure.

The base unit 310 may be provided with a compressor installation unit 313 in which the compressor 342 configured to supply the refrigerant to the heat exchangers 341 and 343 is installed. The compressor installation unit 313 may be disposed outside the circulation duct 320.

In addition, a controller or a control panel C that controls the clothing treatment apparatus of the present disclosure may be installed on the base unit 310.

The base unit 310 may be provided with a controller installation unit 313 that forms a space into which the controller C is inserted below the circulation duct 320.

The controller C may be provided to control all electronically controlled components, such as the compressor 342, the steam supply 800, and the blower fan 353.

Since the controller C is inserted into the base unit 310 to be supported thereby, and vibration or shock applied to the controller C may be buffered. In addition, since the controller C is disposed close to all electronic components, occurrence of control errors, such as noise, may be minimized.

In addition, the steam supply is disposed above the circulation duct 320, and the controller C is disposed below the circulation duct 320. Therefore, the circulation duct 320 may be provided in a straight duct shape between the steam supply 800 and the controller C. Accordingly, the flow resistance of air passing through the circulation duct 320 may be minimized.

The circulation duct 320, the external air duct 370, the steal supply 800, the controller C, and the steam supply 340 may be provided in a module format on the base unit 310.

Thereby, the base unit 310 may be easily installed and maintained while being withdrawn from or inserted into the machine compartment 300 forwards or rearwards.

FIG. 5(a) is a perspective view of the base unit 310 viewed from the front, and FIGs. 5(b) and 5(c) are perspective views of the base unit 310 viewed from the rear.

The base unit 310 may be installed on a base plate forming the lower surface of the clothing treatment apparatus. The base unit 310 may itself form the lower surface of the clothing treatment apparatus.

The base unit 310 may include a base bottom 311 forming a support surface. The base bottom 311 may form the lower surface of the clothing treatment apparatus. Further, the base bottom 311 may be installed on the upper surface of the bottom surface of the cabinet 100 forming the lower surface of the clothing treatment apparatus.

The base unit 310 may be provided integrally with the circulation duct 320 forming at least a portion of a flow path in which air flows. The circulation duct 320 may be formed to extend upward from the base bottom 311.

The circulation duct 320 may include a duct body 321 that extends from the base bottom 311 to form the flow path, a heat exchanger installation unit 3212 that provides a space in which an evaporator 341 or a condenser 343 is installed in the duct body 321, and an air discharge unit 323 that is provided at the rear of the duct body 321 and through which air inside the duct body 321 is discharged.

The air discharge unit 323 may be provided in a pipe shape that extends rearward from the duct body 321. The diameter of the air discharge unit 323 may be smaller than the width of the duct body 321.

The air discharge unit 323 may be connected to the fan housing 350. The air discharged from the air discharge unit 323 may be guided into the inner case 200 through the fan housing 350.

The circulation duct 320 may include an external air intake part 322 formed to penetrate the front surface of the duct body 321.

The external air intake part 322 may be provided to communicate with the external air duct 370. The external air duct 370 may be placed in front of the external air intake part 322 to be supported.

The circulation duct 320 may be provided with a damper that opens and closes the external air intake part 322. Inflow of external air into the circulation duct 320 may be allowed or blocked by opening and closing the damper.

The base unit 310 may include a compressor installation unit 312 that provides a space in which the compressor 342 is installed. The compressor installation unit 312 may be formed on one side of the base bottom 311, and may be formed integrally with the base bottom 311.

The compressor installation unit 312 may have a protrusion that supports the compressor 342. The compressor installation unit 312 may be disposed on the rear portion of the base unit 310. The compressor installation unit 312 may be disposed such that at least a portion thereof overlaps the air discharge unit 323 in the width direction.

A buffer member that reduces vibration transmitted from the compressor 342 may be installed in the compressor installation unit 312. The buffer member may be fixed to the protrusion.

The base unit 310 may include the controller installation unit 313 in which the controller C is installed. The controller installation unit 313 may be formed between the base bottom 311 and the circulation duct 320. The controller installation unit 313 may be formed between the base bottom 311 and the bottom surface of the circulation duct 320. The controller installation unit 313 may be provided in a duct shape configured such that one of the front portion and the rear portion thereof is open below the circulation duct 320.

The structure of the controller installation unit 313 will be described later.

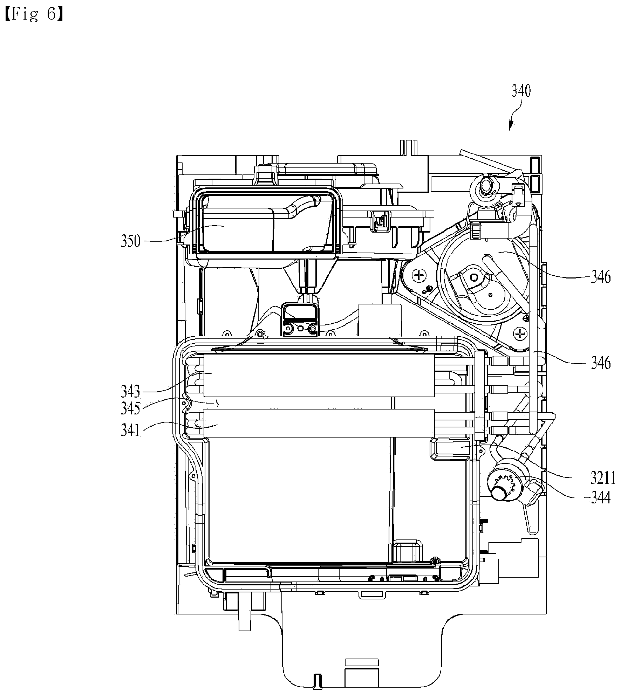

FIG. 6 illustrates the structure of the circulation duct of the clothing treatment apparatus.

The circulation duct 320 may extend upward from the base bottom to form the flow path in which air flows. The circulation duct 320 may include the heat exchanger installation unit 3212 that provides the space in which the evaporator 341 and the condenser 343 are installed. The heat exchanger installation unit 3212 may be provided within the duct body 321.

The duct body 321 may be provided with an open upper surface. The condenser 343 and the evaporator 341 may be inserted through the opening of the duct body 321 to be installed.

The opening of the duct body 321 may be shielded by the base cover 360, and the base cover 360 and the duct body 321 may form the flow path of the circulation duct 320.

The front surface of the duct body 321 may disposed to be spaced apart rearward from the front end of the base bottom 311.

Thereby, the base bottom 311 may secure a support surface 3111 on which one or more of the above-described water supply tank 3 or drain tank 4 and the external air duct 370 are installed and supported.

Meanwhile, the heat supply 340 may include the evaporator 341 provided as a heat exchanger installed in the circulation duct 320 to cool and dehumidify the air introduced into the circulation duct 320, the condenser 343 provided as a heat exchanger configured to heat the air having passed through the evaporator 341 to generate hot air, the compressor 342 configured to supply the refrigerant that exchanges heat with the air to the condenser 343 and disposed outside the circulation duct 320, and an expansion valve 344 configured to expand and cool the refrigerant having passed through the condenser 343.

Further, since the duct body 321 is formed integrally with the base unit 310, the heat exchanger installation unit 3212 may secure a great height, and the heights of the condenser 343 and the evaporator 341 may be increased.

As a result, the widths of the condenser 343 and the evaporator 341 in the forward and rearward directions may be reduced, and thus the number of refrigerant pipes passing through the condenser and the evaporator may be reduced. Accordingly, an effect of reducing the flow loss of air passing through the condenser and evaporator may be exhibited.

Further, the sum of the length of the evaporator 341 and the length of the condenser 343 may be smaller than the length of the heat exchanger installation unit 3212. Accordingly, the length of the heat exchanger installation unit 3212 in the forward and rearward directions may be equal to or smaller than half the length of the duct body 321.

Therefore, since the heat exchanger installation unit 3212 may be sufficiently separated from the external air intake part 322, a sufficient space into which external air and the air inside the inner case 200 are introduced may be secured in the circulation duct 320.

Further, the inside of the duct body 321 may include an installation partition 3211 that separates the heat exchanger installation unit 3212 from the outside of the heat exchanger installation unit 3212. The installation partition 3211 may be provided to protrude from the side surface of the duct body 321 to support the front portion of the evaporator 341.

In addition, the duct body 321 may be expanded in width based on the installation partition 3211, and extend rearward.

As a result, the width of the heat exchanger installation unit 3212 may be greater than half the width of the base unit 310. In addition, the width of the circulation duct 320 may be greater than half the width of the base unit 310.

The width of the condenser 343 and the width of the evaporator 341 may also be greater than half the entire width of the base unit 310.

When the widths of the condenser 343 and the evaporator 341 are secured, as described above, there may be an effect of sufficiently securing a heat exchange capacity.

In addition, the fan housing 350 may be disposed to overlap the condenser 343 or the evaporator 341 in the forward and rearward directions. Accordingly, air having passed through the condenser 343 and the evaporator 341 may be introduced into the fan housing 350 without bending the flow path. That is, the air introduced into the circulation duct 320 has an effect of minimizing flow loss because the flow path is not bent during the process of moving the air to the fan housing.

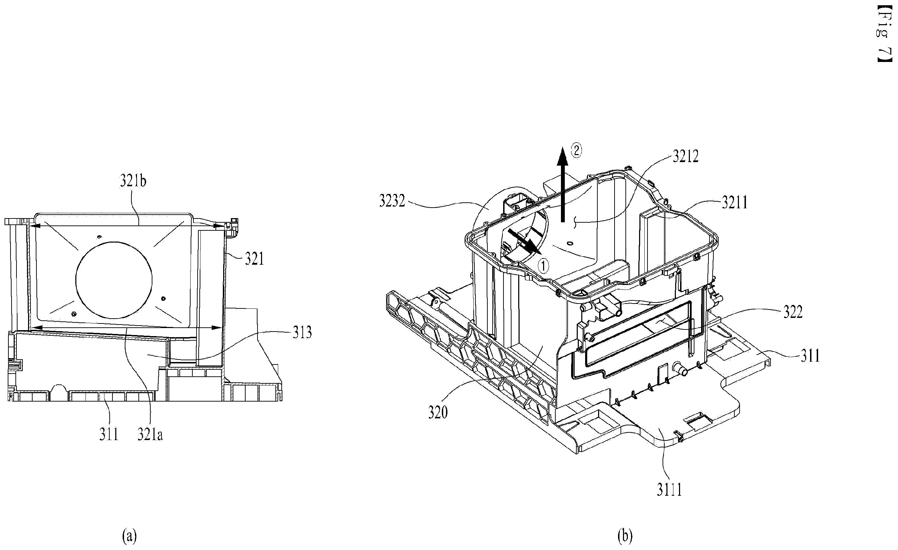

FIG. 7 illustrates the shape of the circulation duct of the clothing treatment apparatus of the present disclosure.

The base bottom 310 and the circulation duct 320 of the base unit 310 may be formed as a single body by injection molding.

A mold configured to form the inner surface of the duct body 321 may be withdrawn upward from the inside of the duct body 21 to be removed. Here, in order to facilitate withdrawal of the mold, the wall surface of the duct body 321 may be inclined at a predetermined angle with respect to the removal direction of the mold.

The width of a lower surface 321a of the duct body 321 may be longer than the width of an upper surface 321b of the duct body 321.

Specifically, a distance between the wall surfaces of the duct body 321 facing each other may increase as the duct body gets farther from the base bottom 311. A distance between the left and right surfaces of the circulation duct facing each other may increase in the withdrawal direction of the mold. Accordingly, the mold may be easily removed.

Meanwhile, the discharge unit 323 may include an air extension pipe 3231 that extend from the rear portion of the duct body 321 to have a reduced diameter or width, and an air discharge pipe 3232 that extends from the air extension pipe 3231 in a pipe shape having a uniform diameter to form a hollow 3233 formed therein. The air extension pipe 3231 may function as a nozzle and thus increase the rate of discharged air.

In addition, a mold for forming the air discharge unit 323 may be removed a shown in the figure above. The mold may be withdrawn forward from the inside of the air discharge unit 323 toward the inside of the circulation duct 320, and then be removed toward the open upper surface of the circulation duct 320. In this process, the air discharge unit 323 may be formed in a structure that facilitates withdrawal of the mold.

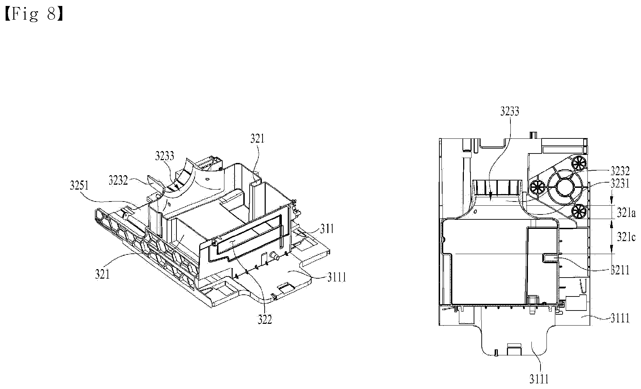

FIG. 8 is a cross-sectional view of the circulation duct.

The installation partition 3211 may protrude inward from the inner wall of the duct body 321, or be formed by indenting the outer wall of the circulation duct inward.

The heat exchanger installation unit 3212 may be formed between the heat exchanger installation partition 3211 and the air discharge unit 323.

The mold for forming the air discharge unit 323 may be withdrawn forward from the air discharge unit 323 and then withdrawn upward to be removed. It is necessary to prevent the mold for forming the air discharge unit 323 from interfering with the heat exchanger installation partition when the mold is withdrawn forward from the inside of the air discharge unit 323. For this purpose, the design values of the air discharge unit 323 may be adjusted.

Specifically, when forming the air discharge unit 323, a mold for forming the front portion of the air discharge unit 323 and a mold for forming the rear portion of the air discharge unit 323 based on a parting line 3233 of the air discharge unit 323 of this figure may be separately provided. Accordingly, the removal directions of the molds may be different from each other. The mold for forming the front portion of the air discharge unit 323 based on the parting line of the air discharge unit 323 may be withdrawn forward, and the mold for forming the rear portion of the air discharge unit 323 based on the parting line of the air discharge unit 323 may be withdrawn rearward.

That is, in order to prevent the mold withdrawn forward from interfering with the heat exchanger installation partition during the withdrawal process, a distance 1 321a may be smaller than a distance 2 321c in the figure. The distance 1 321a may mean a distance between the parting line of the air discharge unit 323 and the front end of the air discharge unit 323. In addition, the distance 1 321a may mean a distance between the parting line of the air discharge unit 323 and the rear opening of the circulation duct. The distance 2 321c may mean a distance between the front end of the air discharge unit 323 and the heat exchanger installation partition. In addition, the distance 2 323c may mean a distance between the rear opening of the circulation duct and the heat exchanger installation partition 3211.

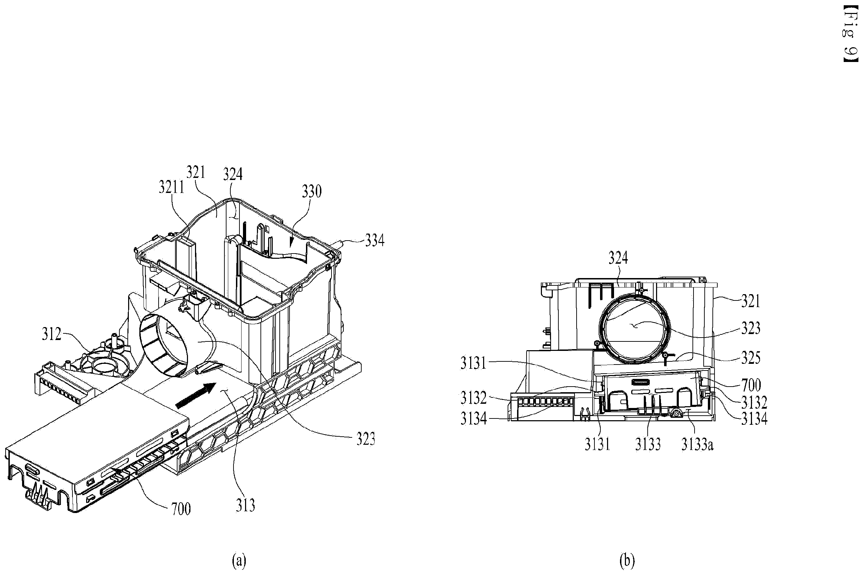

FIG. 9 illustrates the structure of the controller installation unit provided on the base unit of the clothing treatment apparatus of the present disclosure.

FIG. 9(a) illustrates an embodiment in which the controller C is installed in the controller installation unit 313.

The controller C may be provided so that the clothing treatment apparatus of the present disclosure is capable of controlling all devices necessary to perform an arbitrary course for performing the refreshing cycle on clothes. The controller C may be provided as a PCB substrate, but is not limited thereto, and may be provided as various devices for control.

The controller C may be inserted into the controller installation unit 313 to be placed therein.

The controller installation unit 313 may be disposed below the circulation duct 320.

The bottom surface of the circulation duct 320 may form the upper surface of the controller installation unit 313. The controller installation unit 313 may be disposed below the air discharge unit 323.

The controller installation unit 313 may be formed integrally with the base bottom 311. The controller installation unit 313 may be formed as a sunken space under the circulation duct during a process of molding the circulation duct 320 in the base unit 310.

The controller C may be introduced into the controller installation unit 313 forward from the rear in a sliding manner.

Brackets 3131 provided to surround the controller may be provided on the surface of the controller C. The brackets 3131 may be disposed on the upper and lower portions of the controller to prevent foreign substances from entering the controller.

In addition, the brackets 3131 may prevent damage to a circuit board in the controller C due to heat or vibration transmitted to the controller C. The brackets 3131 may be formed of a metal material.

FIG. 9(b) illustrates a state in which the controller is installed in the controller installation unit.

As shown in this figure, the controller C may be installed at a predetermined angle with the base bottom 311.

For example, the controller C may be disposed to be inclined toward a water reservoir 326. Accordingly, if water leaks to the upper portion of the controller C, the water may quickly escape the controller C, and the bottom surface of the circulation duct 320 may be molded to be inclined toward the water reservoir 326.

The controller C may include supporters 3132 that are formed to protrude from the side surface of the controller C.

The controller installation unit 313 may include ribs 3134 that protrude from both side surfaces of the installation unit. The supporters 3132 of the controller may be held on the upper portions of the ribs 3134.

The supporters 3132 of the controller may support the entire load of the controller C. When the supporters 3132 of the controller are supported by the upper surfaces of the ribs 3134, the controller C may be spaced apart from the base bottom 311 by a predetermined distance.

The ribs 3134 may be formed integrally with the base unit 310. The ribs 3134 may be formed together with the base unit 310 when the base unit 310 is injection-molded, and may be provided integrally with the base bottom 311, the circulation duct 320, etc.

A protrusion 3133 formed to protrude may be provided on the front surface of the controller C. In addition, a guide that protrudes rearward may be provided on the inner surface of the controller installation unit 313. The protrusion may be coupled to the guide. The protrusion may be inserted into the guide. When the controller is inserted into the controller installation unit, the controller may be aligned in the correct position by coupling the protrusion to the guide.

In addition, the positions of both side surfaces of the controller may be determined in the above-described manner in which the supporters are placed on the ribs. The controller may be coupled to the correct position of the controller installation unit without a separate fastening member using the above coupling process.

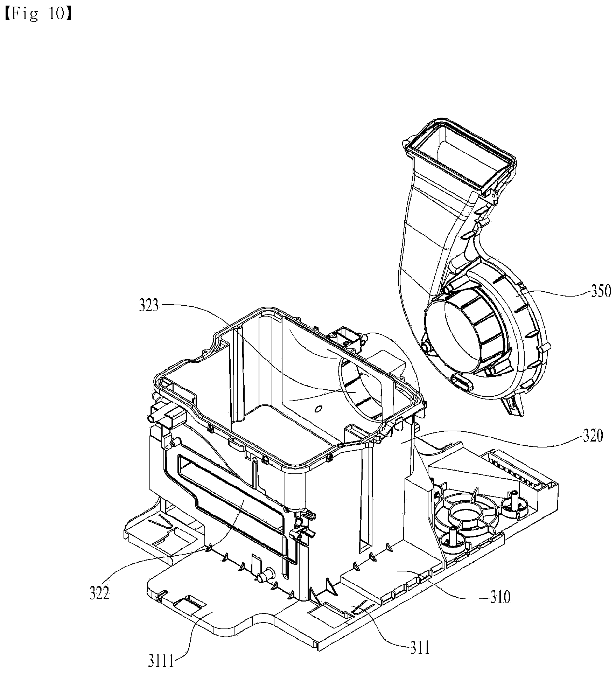

FIG. 10 illustrates the structure of the air discharge unit 323 of the clothing treatment apparatus of the present disclosure.

The base unit 310 may include the air discharge unit 323 that discharges treated air toward the fan housing.

The air discharge unit 323 may be provided to allow the fan housing 350 to communicate with the inside of the circulation duct 320 or the duct body 321. The air discharge unit 323 may be provided in a bell mouth shape. The air discharge unit 323 provided in the bell mouth shape may reduce air flow loss and improve air circulation efficiency.

The air discharge pipe 3232 of the air discharge unit 323 may be provided in a pipe shape, and during the mold removal process based on the parting line 3233, the mold disposed in front of the parting line 3233 may be withdrawn forward and the mold disposed at the rear of the parting line 3233 may be withdrawn rearward.

The fan installation unit 350 may be coupled to the air discharge pipe 3232 to be supported thereby. The fan housing 351 may have a coupling hole coupled to the outer circumferential surface of the air discharge pipe 3232, and the blower fan 353 may be disposed in the coupling hole.

The fan housing 351 may include the discharge duct 352 that extends from the outer circumferential surface of the blower fan 353 or the outside to the discharge hole 232.

The fan housing 351 and the discharge duct 352 may form a flow path in which the blower fan 353 is accommodated and air moves.

A motor that rotates the blower fan 353 may be coupled to the outside of the fan housing 351 to be supported thereby.

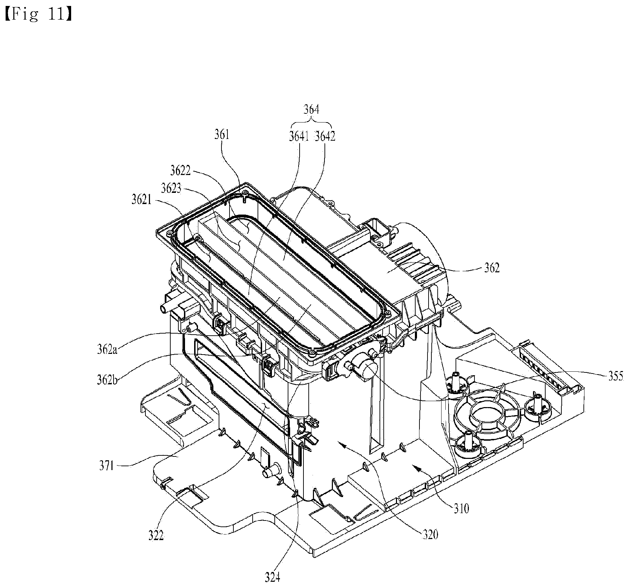

FIG. 11 illustrates the structure of the base cover of the clothing treatment apparatus of the present disclosure.

The base cover 360 may be provided to be coupled to the upper surface of the circulation duct 320 so as to prevent the inside of the circulation duct 320 from being exposed.

The base cover 360 may include an inflow body 361 coupled to the upper surface of the circulation duct 320 to allow the inner case 200 to communicate with the circulation duct 320, and a shielding body 363 extending from the inflow body 361 to shield the circulation duct 320.

The inflow body 361 may be provided in a duct shape to allow the inflow hole 231 of the inner case to communicate with the inside of the circulation duct 320. The inflow body 361 may be provided to protrude farther upward than the shielding body 363.

The inflow body 361 may be disposed ahead of the evaporator 341 so as not to face the evaporator 341 and the condenser 343, and may be disposed ahead of the partition 3211.

The inflow body 361 may serve as an inflow duct that moves air of the inner case 200 to the circulation duct 320.

The inflow body 361 may be provided with the inflow part 362 through which the air of the inner case 200 may pass.

Specifically, the base cover 360 may include a first rib 362a extending in the width direction of the inflow body 361, and a second rib 362b spaced apart rearward from the first rib 362a and extending in the width direction of the inflow body 361.

The first rib 362a and the second rib 362b may be provided in parallel. The first rib 362a and the second rib 362b may be provided in a plate shape extending in the vertical direction, and the height thereof may correspond to the height of the inflow body 361.

The front surface of the inflow body 361 and the first rib 362a may form a first inlet 3621, the first rib 362a and the second rib 362b may form a second inlet 3623, and the second rib 362b and the rear surface of the inflow body 361 may form a third inlet 3622.

The first inlet 3621 and the third inlet 3622 may be provided with the same area, and the second inlet 36222 may be provided with a smaller area than the first inlet 3621 and the third inlet 3622.

The base cover 360 may include a damper unit 364 provided to open and close the inflow part 362, and a driver 365 coupled to the damper unit 364 to control opening and closing of the damper unit 364.

The damper unit 364 may include a first damper 3641 provided to open and close the first inlet 3621, and a second damper 3642 provided to open and close the third inlet 3622.

The first damper 3641 may be provided in a plate shape with an area corresponding to the first inlet 3621, and may be rotatably coupled to both side surfaces of the inflow body 361 within the first inlet 3621.

The second damper 3642 may be provided in a plate shape with an area corresponding to the third inlet 3622, and may be rotatably coupled to both side surfaces of the inflow body 361 within the third inlet 3622.

The second inlet 3623 may be provided with a cut-off filter 366 that allows air to pass therethrough but may filter out foreign substances, such as fine dust and lint.

The cut-off filter 366 may be provided to be inserted into the second inlet 3623 to divide the first inlet 3621 and the third inlet 3622. The cut-off filter 366 may be disposed to extend from the second inlet 3623 to come into contact with the bottom surface of the circulation duct 320.

The cut-off filter 366 may be provided as a filter capable of filtering moisture. For example, the cut-off filter 366 may be provided as a HEPA filter, or the like.

A shielding member that shields the second inlet 3623 when the cut-off filter 366 is inserted thereinto may be further coupled to the second inlet 3623.

The driver 365 may include a motor that provides power to selectively rotate the first damper 364 and the second damper 365, and a plurality of gear members that is engaged with the motor and rotates to selectively rotate the first damper 364 and the second damper 365.

The first inlet 3611 and the third inlet 3622 may be selectively opened due to the driver 365.

Due to the driver 365, air accommodated in the inner case 200 may be introduced into the circulation duct 320 along the first inlet 3621, or may be introduced into the circulation duct 320 along the third inlet 3622.

Of course, the driver 365 may control the first damper 3641 and the second damper 3642 to open both the first inlet 3611 and the third inlet 3622, and may control the first damper 3641 and the second damper 3642 to shield both the first inlet 3611 and the third inlet 3622.

The driver 365 may be provided in any structure as long as it may be provided to rotate the first damper 3641 and the second damper 3642. For example, the driver 365 may be provided as a combination of a motor, a driving gear rotated by the motor, and driven gears coupled to the first damper and the second damper and rotated due to rotation of the driving gear.

The base cover 360 may include the shielding body 363 that extends from the inflow body 361 and is capable of shield the evaporator 341 and the condenser 343. The shielding body 363 may be provided in a plate shape.

The base cover 360 may be detachably coupled to the upper surface of the circulation duct 320 through an inflow hook 3612 extending from the lower surface of the inflow body 361.

The circulation duct 320 may be provided with a coupling part detachably coupled to the inflow hook 3612.

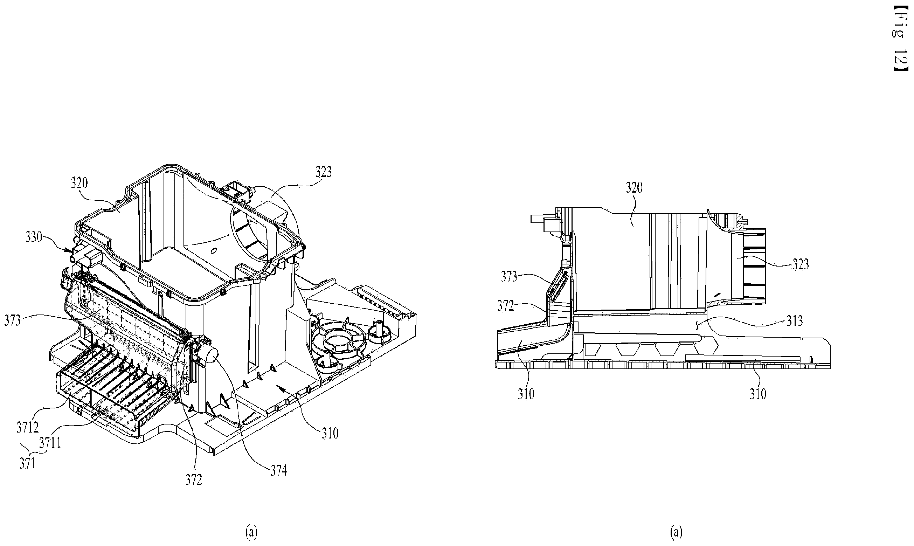

FIG. 12 illustrates the structure of the external air duct.

Referring to FIG. 12(a), the external air duct 370 may be coupled to the base unit 310.

The external air duct 370 may be provided to communicate with the external air intake part 322.

The external air duct 370 may include an external air damper 373 that opens and closes the external air intake part 322, and an external air driver 374 that rotates the external air damper 373 to selectively open the external air intake part 322.

The external air damper 373 may be provided in a plate shape that may seal the external air intake part 322, and may be rotatably coupled to both side surfaces of the external air intake part 322.

The external air driver 374 may be provided as an actuator coupled to the external air duct 370 or the circulation duct 320 to rotate the external air damper 373.

The outdoor air duct 370 may include an extension duct 372 that extends forward from the external air intake part 322 in front of the external air intake part 322, and an intake duct 371 that extends forward from the extension duct 372 and allows external air to be introduced.

The intake duct 371 may be provided to extend from the lower portion of the extension duct 372, and the water supply tank 3 and the drain tank 4 may be disposed on the upper portion of the intake duct 371. The water supply tank 3 and the drain tank 4 may be coupled to or placed on the intake duct 371.

The intake duct 371 may include an external air hole 3711 formed at one end or a free end thereof so that external air is suctioned through the external air hole 3711, and a partition rib 3712 provided to partition the external air hole 3711.

The external air hole 3711 may be provided to be disposed below the door 40 so as not to be shielded by the door 40.

The partition rib 3712 may be provided to partition the inside of the external air hole 3711 so as to prevent foreign substances or a user's body from being inserted into the external air hole 3711.

Referring to FIG. 12(b), when the external air driver 374 rotates the external air damper 373 to open the external air intake part 322, the intake duct 371 and the circulation duct 320 may communicate with each other.

At this time, when the blower fan 352 is operated, air outside the cabinet may be introduced into the circulation duct 320. When the compressor 342 is operated, the external air may be dehumidified while passing through the circulation duct 320, and be supplied to the inside of the inner case 200.

The door 40 may further include a discharge hole through which air inside the inner case 200 is discharged to the outside, and a discharge damper that selectively opens and closes the discharge hole. The discharge hole may be provided to face the accommodation space of the inner case 200.

Thereby, the dehumidified air may be discharged through the discharge hole.

In addition, the external air may be filtered while passing through the cut-off filter 366, and be discharged again to the outside of the cabinet 100.

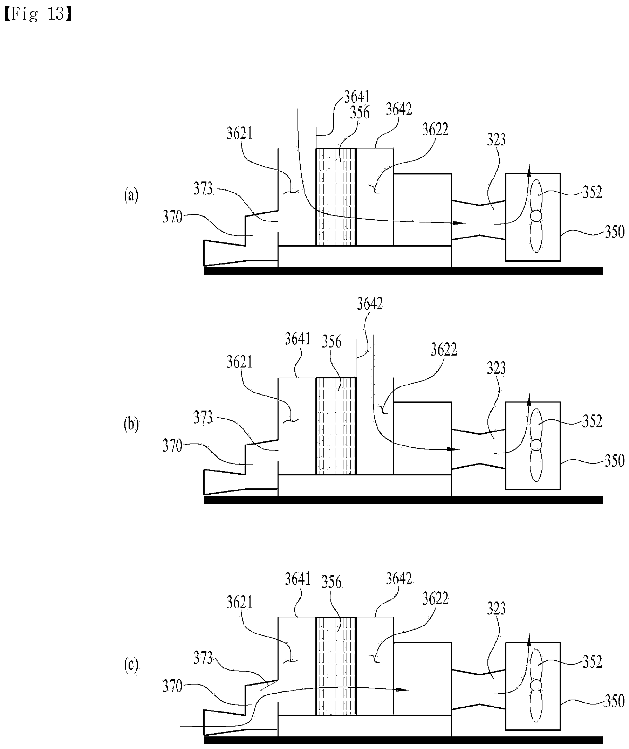

FIG. 13 illustrates the flow of air flowing through the circulation duct.

Referring to FIG. 13(a), the external air damper 373 may be controlled to shield the external air intake part 322, the first damper 3641 may be controlled to open the first inlet 3621, and the second damper 3642 may be controlled to shield the third inlet 3622.

When the blower fan 352 is operated, the air inside the inner case 200 may be introduced into the first inlet 3621 to be filtered while passing through the cut-off filter 366.

When the compressor 342 is operated, the air having passed through the cut-off filter 366 may be dehumidified and heated while passing through the evaporator 341 and the condenser 343.

The air having passed through the heat exchangers may pass through the fan installation unit 350 and be supplied to the inside of the inner case 200.

This state may be a state in which steam is not supplied to the inner case 200. This is because, if steam is supplied to the inner case 200, the moisture wets the cut-off filter 366, and the performance of the cut-off filter 366 may not be guaranteed.

As a result, in a state in which steam is not supplied to the inner case 200, before steam is supplied to the inside of the inner case 200, or if the humidity is low even after steam is supplied to the inside of the inner case 200, the air inside the inner case 200 may pass through the first inlet 3641 and the cut-off filter 366 to filter out foreign substances, such as lint.

Referring to FIG. 13(b), the external air damper 373 may be controlled to shield the external air intake part 322, the first damper 3641 may be controlled to shield the first inlet 3621, and the second damper 3642 may be controlled to open the third inlet 3622.

When the blower fan 352 is operated, the air inside the inner case 200 may be introduced into the third inlet 3622. Since the third inlet 3622 is provided downstream from the cut-off filter 366, the air introduced into the third inlet 3622 may not pass through the cut-off filter 366.

When the compressor 342 is operated, the air having passed through the cut-off filter 366 may be dehumidified and heated while passing through the evaporator 341 and the condenser 343.

The air having passed through the heat exchangers may pass through the fan installation unit 350 and be supplied to the inside of the inner case 200.

As a result, in a state in which steam is supplied to the inner case 200 or if the humidity inside the inner case 200 is very high, the air inside the inner case 200 may be introduced into the third inlet 3622, and be prevented from being introduced into the first inlet 3621, thereby preventing the cut-off filter 366 from being exposed to moisture.

Referring to FIG. 13(c), the external air damper 373 may be controlled to open the external air intake part 322, the first damper 3641 may be controlled to shield the first inlet 3621, and the second damper 3642 may be controlled to shield the third inlet 3622.

When the blower fan 352 is operated, the air inside the inner case 200 may be prevented from being introduced into the inflow part 362, and only the air outside the cabinet 100 may be introduced into the circulation duct 320 and pass through the cut-off filter 366. Thereby, foreign substances, such as fine dust, contained in the external air may be filtered out by the cut-off filter 366.

When the compressor 342 is operated, the air having passed through the cut-off filter 366 may be dehumidified and heated while passing through the evaporator 341 and the condenser 343.

The air having passed through the heat exchangers may pass through the fan installation unit 350 and be supplied to the inside of the inner case 200, thereby supplying fresh hot air to clothes.

At this time, if a device for discharging the air inside the inner case 200 to the outside is provided, the air outside the cabinet may be discharged in a purified and dehumidified state while passing through the cut-off filter 366 and the heat supply 340.

As a result, the clothing treatment apparatus of the present disclosure may determine the flow directions of the air inside the inner case 200 and the air outside the cabinet by controlling the external air driver 374 and the inflow drier 365 through the controller C.

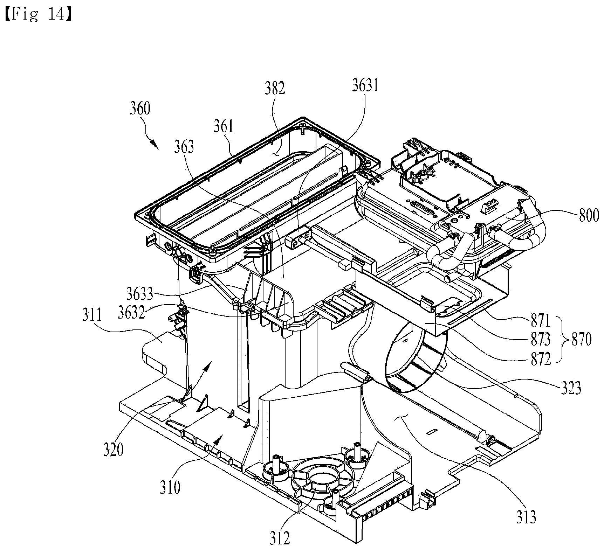

FIG. 14 illustrates the installation structure of the steam supply.

The steam supply 800 may be placed on the base cover 360 to be supported thereby.

The steam supply 800 may include a steam case 810 that is placed on the base cover 360 and stores water to generate steam.

The steam supply 800 may further include an installation bracket 870 that may fix the steam case 810 to the base cover 360.

The installation bracket 870 may be coupled to the base cover 360 to fix the steam case 810.

The installation bracket 870 may include a lower panel 871 that supports the lower surface of the steam case 810, and side panels 872 that support both side surfaces of the steam case 810 on the lower panel 871.

The installation bracket 870 may further include one or more fixing clips 873 that extend from the side panels 872 to prevent the steam case 810 from being detached.

The fixing clips 873 may be detachably provided on the upper portion or the side surfaces of the steam case 810.

The compressor 342 may be disposed below the steam supply 800.

The installation bracket 870 may be provided to block transfer of heat generated from the compressor or heat generated from the refrigerant compressed by the compressor to the steam supply 800.

The installation bracket 870 may also block transfer of fire to the steam supply 800 in the event of a fire occurring in the compressor 342.

Meanwhile, the base cover 360 may include a fastener 3631 that is provided on the shielding body 360 and detachably coupled to the steam supply 800. The fastener 3631 may be provided in a structure that is detachably coupled to a protrusion protruding from the lower portion of the steam case 810.

Accordingly, even if a large amount of water is accommodated in the steam case 810, the steam case 810 may be stably placed on the base cover 360.

In addition, since the steam case 810 is disposed above the circulation duct 320 and a distance from the inner case 200 is shorter, condensation of steam generated in the steam case 810 before reaching the inner case 200 may be minimized.

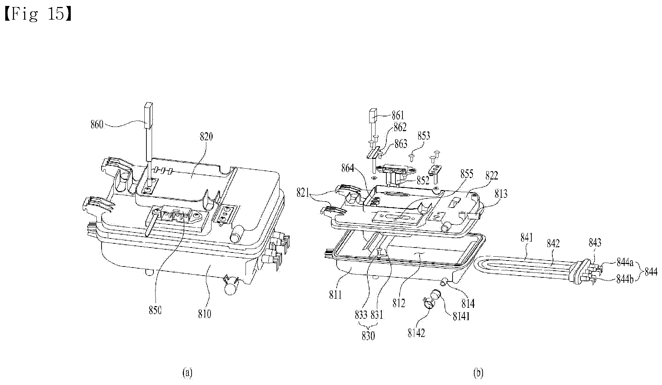

FIG. 15 illustrates the detailed structure of the steam supply.

Referring to FIG. 15(a), the steam supply 800 may include the steam case 810 that may receive and store water to generate steam, and a heater unit 840 accommodated in the steam case 810 to heat water to generate steam.

The steam case 810 may be provided in the form of a case with an open upper portion to accommodate the heater unit 840.

The steam supply 800 may further include a case cover 820 coupled to the steam case 810 to prevent the heater unit 840 from being exposed to the outside and prevent the water from leaking.

A water level sensor 850 that detects the water level of the steam case 810 and a steam sensor 60 that detects the temperature inside the steam case 810 or detects whether steam is generated in the steam case 810 may be installed on the case cover 820.

Referring to FIG. 15(b), the steam case 810 may include a case body 811 that provides a space configured to store the water and accommodate the heater unit 840.

The case body 811 is provided with an open upper portion so that various parts may be easily installed in the case body 811.

The case body 811 may include a heater insertion hole 8111 formed through one side thereof so that the heater unit 840 may be inserted into or withdrawn from the heat insertion hole 8111.

The case body 811 may include a recovery pipe 814 configured to discharge water accommodated in the case body 811 to the outside.

The recovery pipe 814 may be kept closed by a shielding plug 8141 so as to be opened only when removing residual water in the steam case 810, and may include a shielding clip 8142 that maintains the coupled state of the shielding plug 8141 to the recovery pipe 814 to prevent the shielding plug 8141 from being separated arbitrarily.

Accordingly, when repairing the steam case 800 or preventing freezing of the steam case 800, water inside the steam case 800 may be discharged through the recovery pipe 814.

Meanwhile, a heater fixing unit 830 that may support or fix the heater unit 840 may be installed in the case body 811. The heater fixing unit 830 may include a fixing clip 831 that fixes the heater unit 840, and a clip fastening member 833 that fixes the fixing clip 831 to the case body 811.

The fixing clip 831 may be provided to accommodate or surround at least a portion of the heater unit 840.

The steam supply 800 may be provided with a water supply pipe 815 that supplies water. The water supply pipe 815 may be provided to communicate with the water supply tank 30 to receive water therefrom.

The water supply pipe 815 may be provided on the case cover 820, or may be disposed on the upper portion of the steam case 810. Accordingly, counterflow of water through the water supply pipe 815 may be prevented.

The steam supply 800 may be provided with a steam pipe 813 that discharges steam generated by operation of the heater unit 840 to the outside. The steam pipe 813 may be provided on the upper portion of the case cover 820 to prevent water from being discharge into the steam pipe 813. The steam pipe 813 may communicate with the steam hole 233 of the inner case 200.

The case cover 820 may be provided with a water level sensor hole 854 in which the water level sensor may be installed.

The water level sensor 850 may include one or more contact protrusions 852 that are inserted into the water level sensor hole 854 and immersed in water to detect the water level, and a sensor body 851 that is coupled to the water level sensor hole 854 or supported by the case cover 820 to maintain the contact protrusions 852 in a floating state within the steam case 810.

The sensor body 851 may be coupled to the case cover 820 through a sensor fastening member 853.

Meanwhile, the case cover 820 may be provided with an insertion hole 64 in which the steam sensor 860 may be installed. The steam sensor 860 may include a detection device 861 inserted into the insertion hole 864 to detect whether steam is generated in the steam case 810, a support 863 that fixes the detection device 861 to the case cover 20, and a coupling member 862 that couples the support 863 to the case cover 820.

The detection device 861 may be provided as a humidity sensor or a temperature sensor to detect whether steam is generated in the steam case 810.

Further, the case cover 820 may be provided with cover hooks 821 that may extend forward and be coupled to the base cover 860.

In addition, fixing protrusions 822 that may fix the lower portion of the inner case 200 or a separate discharge unit 900 may be provided at the rear portion of the case cover 820.

The heater unit 840 may be inserted into the heater insertion hole 8111 to be accommodated in the steam case 810, and be provided to receive power to heat water.

The heater unit 840 may be provided as a sheath heater, etc., and may be controlled by a controller 700 to be repeatedly operated and stopped.

The heater unit 840 may include a first heater 841 that receives first power to heat water, and a second heater 842 that receives power greater than the first power to heat water.

As a result, the second heater 842 may be provided to heat a larger amount of water than the first heater 841 to generate a larger amount of steam.

The first heater 841 and the second heater 842 may be provided to consume respective power amounts divided from the maximum heater power amount allowed for the heater unit 840. That is, if the first heater 841 is provided to consume a portion of the maximum heater power amount, the second heater 842 may be provided to consume the remainder of the maximum heater power amount.

For example, if the maximum heater power amount generally allowed for the heater unit 840 is 1,500 W, the first heater 841 may be provided to consume 600 W, and the second heater 842 may be provided to consume 880 W. 20 W may be left considering errors, etc.

Of course, the heater unit 840 may include three or more heaters. For example, the heater unit 840 may include the first heater 841, the second heater 842, and a third heater 843, and the first heater 841, the second heater 842, and the third heater 843 may be provided to consume respective power amounts divided from the maximum heater power amount.

Hereinafter, the heater unit 340 will be described as including the first heater 841 and the second heater 842.

The first heater 841 and the second heater 842 may be formed as a U-shaped metal pipe.

The heater unit 840 may include a heater sealer 843 that may fix the first heater 841 and the second heater 842 and seal the heater insertion hole 8111, and may include a terminal unit 844 that supplies current to the first heater 841 and the second heater 842.

The terminal unit 844 may include a first terminal 844a that supplies current to the first heater 841, and a second terminal 844b that supplies current to the second heater 842.

The first heater 841 and the second heater 842 may be disposed at the same height. Therefore, the first heater 841 and the second heater 842 may be provided to heat water of the same water level to generate steam.

Accordingly, the controller 700 may control a steam amount generated using both or selectively the first heater 841 and the second heater 842, and a power amount consumed.

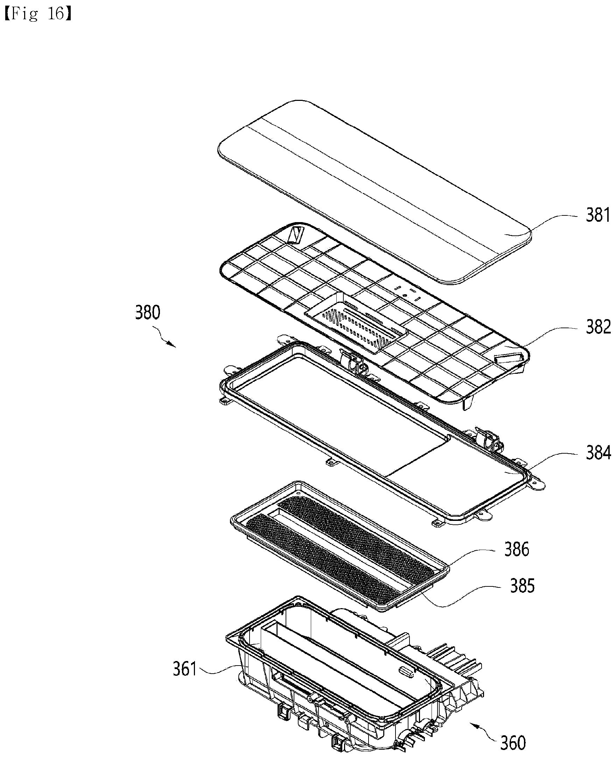

FIG. 16 illustrates a filter unit of the clothing treatment apparatus of the present disclosure.

The clothing treatment apparatus 1 may further include a filter unit 380 in the upper portion of the machine compartment 300 to prevent dust from entering the inside of the circulation duct 320.

The filter unit 380 may be mounted on the inflow body 361 to filter out dust introduced into the inflow body 361.

The filter unit 380 may include a body filter 386 mounted on the upper portion of the inflow body 361 of the base cover 360 to shield a portion of the upper surface of the inflow body 361. The body filter 386 may be formed of a mesh material that may block large dust particles and allow light to pass therethrough.

The filter unit 380 may further include a body cover 385 on which the body filter 386 is placed. The body cover 385 may be mounted on the upper portion of the inflow body. The body cover 385 may shield one surface of each of the upper portions of the first inlet 3621 and the third inlet 3622. Portions of the body cover 385 that shield the upper surfaces of the first inlet 3621 and the third inlet 3622 may be provided through perforation. The body cover 385 may block large dust particles when the body filter 386 is not installed, thereby being capable of preventing dust from entering the inside of the circulation duct 320.

The filter unit 380 may further include a filter fixing unit 384 to fix the body cover 385 and the body filter 386. The filter fixing unit 384 may be provided on the upper portion of the base cover 360. The circumferential surface of the body cover 385 may be coupled to the filter fixing unit 384, and may be attached to or detached from the filter fixing unit 384.

The clothing treatment apparatus may further include a filter cover 382 on the upper portion of the body cover 385. The filter cover 382 may be provided to have a larger width than the width of the body cover 385. The filter cover 382 may shield one surface of the body filter 386. The lower surface of the filter cover 382 may be formed as a lattice-shaped injection molded product. The filter cover 382 may be provided to be spaced apart from the bottom surface of the inner case 200 so that air inside the inner case 200 may be introduced into the circulation duct 320.

A blocking cover 381 may be provided on the upper portion of the filter cover 382. The blocking cover 381 may completely shield the open upper surface of the machine compartment 300.

The filter unit 380 may include the blocking cover 386.

Hereinafter, embodiments of the clothing treatment apparatus for determining whether the filter unit 380 is attached or detached will be described. For example, the clothing treatment apparatus of the present disclosure may detect whether the body filter 386 is attached or detached.

There is a problem in that a user erroneously uses the clothing treatment device while the filter is not installed when using the clothing treatment apparatus, and if the filter is not installed, there is a high likelihood of large dust particles entering the inside of the circulation duct 320, which may cause sanitation problems.

Therefore, the clothing treatment apparatus 1 of the present disclosure may include the cabinet 100 having the opening in the front portion thereof, the inner case 200 provided in the cabinet 100 to provide the space configured to accommodate clothes, the door 40 coupled to the cabinet 100 to open and close the opening, the inflow body 361 provided below the bottom surface of the inner case 100 so that air from the inner case 100 is introduced into the inflow body 361, the machine compartment including the discharge duct 352 that discharges air to the inner case 100, the filter unit 380 mounted on the inflow body 361 to filter out dust introduced into the inflow body 361, the light emitting unit 600 disposed to be spaced apart from the filter unit 380 and provided to emit light toward the filter unit 380, and the sensor unit 700 provided to detect the light emitted from the light emitting unit 600 and having passed through the filter unit 380.

The clothing treatment apparatus of the present disclosure may further include the controller C that determines at least one of whether the filter unit 380 is attached or detached or the contamination level of the filter unit 380 depending on the amount of the light emitted from the light emitting unit 600, detected by the sensor unit 700.

The present disclosure provides the clothing treatment apparatus including the light emitting unit 600 and the sensor unit 700 for detecting whether the filter unit 380 is attached or detached using light.

Basically, the positions of the light emitting unit 600 and the sensor unit 700 may be determined so that the filter unit 380 is installed between the light emitting unit 600 and the sensor unit 700.

For example, one of the light emitting unit 600 and the sensor unit 700 may be disposed above the filter unit 380, and the other may be disposed below the filter unit 380.

The clothing treatment apparatus of the present disclosure may determine whether the filter unit 380 is attached or detached from an increase in the illuminance of light detected by the sensor unit 700 when the filter unit 380 is not installed.

The clothing treatment apparatus determines whether the filter is attached or detached using light, and thus has an advantage in that the sensor unit is less affected by shock and vibration of the clothing treatment apparatus and there is no limit to the life of a light source as long as power is supplied.

Furthermore, since the door 40 is closed and the inside of the inner case 200 is blocked from light from the outside during the clothing treatment process, it is possible to easily detect a change in illuminance using light to determine whether the filter is attached or detached.

In addition, the clothing treatment apparatus of the present disclosure may detect a change in the illuminance of light without direct contact of the sensor with the filter to indirectly and quickly determine whether the filter is attached or detached.

Further, the clothing treatment apparatus of the present disclosure may determine whether the filter is attached or detached using detection of a change in the illuminance of light by the sensor unit 700 as long as light emitted from the light emitting unit 600 reaches the sensor unit 700 through the filter unit 380 regardless of where the light emitting unit 600 and the sensor unit 700 are provided.

In addition, the clothing treatment apparatus of the present disclosure may determine the contamination level of the filter unit 380 using light. Since the body filter 386 blocks dust having a large volume generated from the inside the clothing treatment apparatus, a large amount of dust may accumulate on the body filter 386. The clothing treatment apparatus of the present disclosure may determine how much dust has accumulated on the body filter 386 by detecting a gradual change in the illuminance of light depending on the amount of the accumulated dust through the sensor unit 700.

For example, if dust has accumulated to an extent that does not cause a problem in the performance of the body filter 386, the illuminance decreases by about 22% compared to if no dust has accumulated. Therefore, the clothing treatment apparatus 1 or the controller C may determine that the body filter 386 is contaminated when the illuminance decreases by 22% or more.

The clothing treatment apparatus may include the light emitting unit provided to emit light toward the filter unit 380.

The light emitting unit 600 may be provided to emit any wavelength of light as long as the sensor unit 700 can detect the same. For example, the wavelength of the light emitted from the light emitting unit 600 may be in the ultraviolet spectrum, the visible spectrum, or the like.

The wavelength of the light emitted from the light emitting unit 600 is most preferably in the visible spectrum rather than the infrared spectrum, the ultraviolet spectrum, etc. Infrared light may penetrate objects, and thus pass through the body filter 386 even if the body filter 386 is installed, whereas visible light is electromagnetic waves that are visible to the human eye, and is not capable of penetrating objects. In addition, use of visible light is more appropriate for detecting whether the filter is attached or detached, and may protect user eyesight.

The light source of the light emitting unit 600 may be provided as any device that emits light, such as an LED or a light bulb.

The clothing treatment apparatus may include the sensor unit 700 that receives the light emitted from the light emitting unit 600.

The sensor unit 700 may be provided as an illuminance sensor that detects a change in the illuminance of the light emitted from the light emitting unit 600. Illuminance is a measure of brightness, and when a large amount of light is incident, the illuminance increases, and conversely, when a small amount of light is incident, the illuminance decreases.

The sensor unit 700 may detect at least one of whether the light emitted from the light emitting unit 600 is received or the intensity of the light.

The light emitting unit 600 may be provided toward the sensor unit 700 so that the sensor unit 700 may detect light. The light emitting unit 600 may be provided to emit light into an area where the sensor unit 700 detects the light.

The sensor unit 700 may be provided at a position where it is capable of completely receiving the light emitted from the light emitting unit 600. The detailed installation positions of the light emitting unit 600 and the sensor unit 700 will be described later.

At least one light emitting unit 600 and at least one sensor unit 700 may be provided.

If two or more light emitting units 600 are provided, the amount of light received by the sensor unit 700 increases, and thus, it is possible to more accurately determine whether the filter is attached or detached.

For example, if the body filter 386 is installed, the body filter 386 may be disposed at a position that partially blocks the light emitted from the light emitting unit 600. If the body filter 386 is detached, the light blocked by the body filter 386 reaches the sensor unit 700. As a result, the clothing treatment apparatus or the controller C may determine whether the filter is attached or detached through detection of an increase in illuminance.

In addition, the more dust is attached to the body filter 386, the more the illuminance decreases compared to if the body 386 is clean. Therefore, if a certain level or more of contamination occurs, the contamination level of the body filter 386 may be determined from the decreased illuminance of the light detected by the sensor unit 700.

Specifically, the clothing treatment apparatus of the present disclosure may determine whether the filter unit 380 is attached or detached depending on the amount of the light emitted from the light emitting unit 600 detected by the sensor unit 700.

The sensor unit 700 may detect a first value when the filter unit 380 is installed on the inflow body 361, and the controller C may determine that the filter unit 380 is not installed on the inflow body 361 when the sensor unit 700 detects a value greater than the first value.

The controller C may be provided to shut down operation of at least one of a steam generator or the compressor 342 upon determining that the filter 380 is not installed.

The sensor unit 700 may detect a second value when the filter unit 380 is not contaminated. The second value may be set to be smaller than the first value, and the controller C may determine that the filter unit 380 is contaminated when a value smaller than the second value is detected.

The clothing treatment apparatus of the present disclosure may include the display provided on one of the cabinet 100 and the door 40 to display the state of the filter unit 380, and the controller C may be provided to display the contamination information of the filter unit on the display upon determining that the filter unit 380 is contaminated.

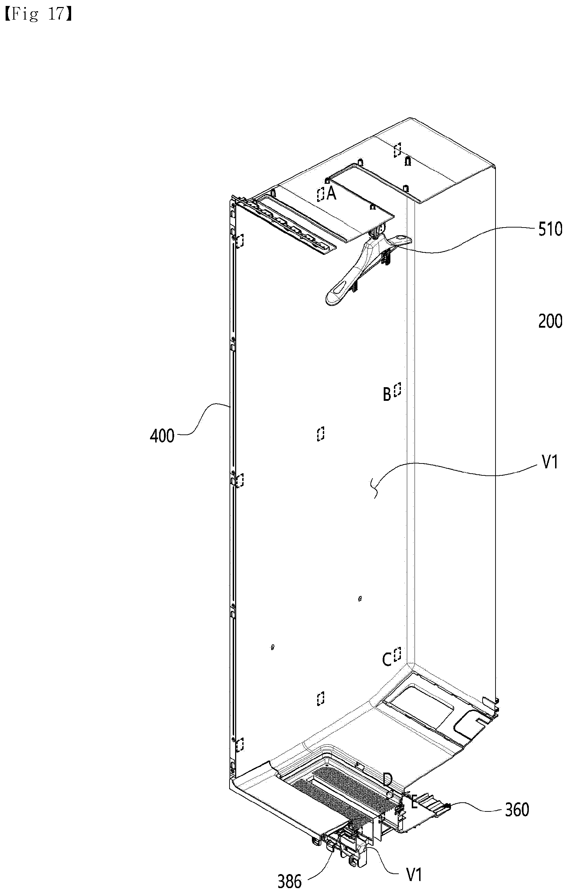

FIG. 17 illustrates position and area division of the clothing treatment apparatus of the present disclosure.

The clothing treatment apparatus 1 may include a first area V1 provided on one side of the filter unit 380 and a second area V2 provided on the other side of the filter unit 380.

The first area V1 and the second area V2 may be divided by the body filter 386 interposed therebetween.

The light emitting unit 600 and the sensor unit 700 may be provided in at least one of the first area V1 or the second area V2.

The first area V1 may be defined as an area including the inner case 200, the upper portion of the machine compartment 300, the upper portion of the body cover 385, etc.

The second area V2 may be defined as an area including the lower portion of the inner case 200, the lower portion of the machine compartment 300, the lower portion of the body cover 385, the inflow body 361, the circulation duct 320, etc.

Meanwhile, the first area V1 and the second area V2 may not be divided into upper and lower parts, left and right parts, etc. based on the body filter 386. It is sufficient to provide the body filter 386 between the first area V1 and the second area V2.

The light emitting unit 600 and the sensor unit 700 may be provided at at least one of positions A, B, C, D, and E. The parts shown in this figure are an example of each position, and the light emitting unit 600 and the sensor unit 700 may be provided at positions not shown in this figure.

The positions A, B, C, and D, may be provided in the first area.

The position A may include an area corresponding to the upper portion of the inner case 200. For example, the position A may be the upper surface of the inner case 200, the lower portion of the hanger unit 510, the hanger unit 510, or the like.

The position B may include several side surfaces excluding the upper surface and the lower surface of the inner case 200 or the inner surface area of the door 40. The position B may be provided at the center of one surface of the inner case 200 in the width direction.

The position C may include an area adjacent to the lower surface of the inner case 200 and the lower portion of the inner case 200. The position C may correspond to an area including one of areas adjacent the lower ends of the side surfaces of the inner case 200 close to the lower surface of the inner case 200.

For example, the position C may be provided at the lower portion of the door 40 close to the lower surface of the inner case 200. In addition, the position C may be the lower surface of the inner case 200.

The position D may include an area that is located below the lower surface of the inner case 200 or the upper surface of the filter unit 380 and above the body filter 386. The position D may include the lower surface of the filter cover 382.

For example, the position D may correspond to the center of the lower surface of the filter cover 382 in the width direction. The position D may be provided at the exact center of the filter cover 382. The position D may be provided one surface above the body cover 385. The position D may be provided on the front surface or the rear surface of the body cover 385.

The position E may include an area below the body filter 386.

For example, the position E may be disposed in the inflow duct of the circulation duct 320.

In addition, the position E may be provided on one surface of the inflow body 361. The position E may be provided in at least one of the first inlet 3621, the second inlet 3622, or the third inlet 3623. The position E may be provided on the front surface or the rear surface of the inflow body 361. The position E may be provided on one surface of the inflow body 361 extending from at least one of the first rib 362a or the second rib 362b of the inflow body 361. The position E may be provided on the lower surface of the perforated plate of the body cover 385 and one surface of the inflow body 361 extending from at least one of the first rib 362a or the second rib 362b.

If the light emitting unit 600 is provided at the position E, deterioration of the performance of the light emitting unit 600 due to dust, etc. filtered out by the body filter 386 may be prevented, and exposure of the user's eyes to light may be prevented. Further, power supply and repair may be facilitated, and influence of clothes hanging in the inner case 200 may be minimized.

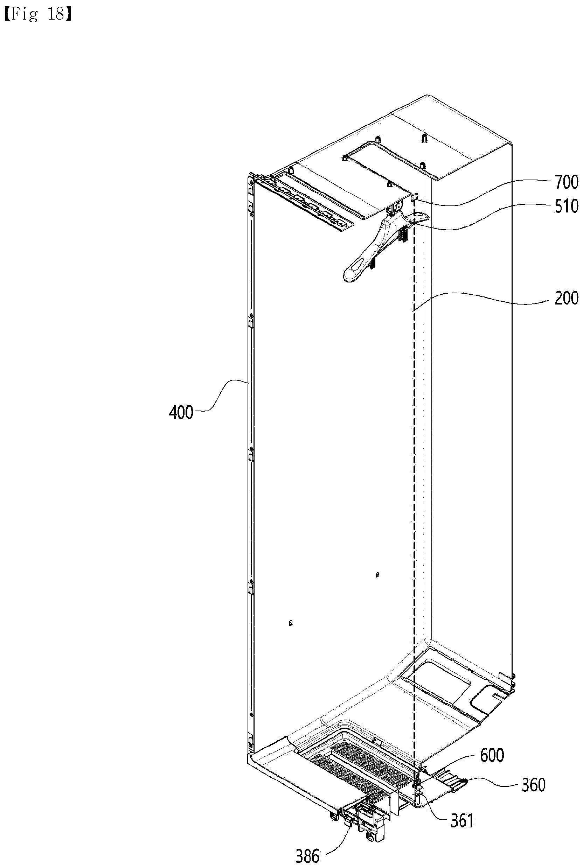

FIG. 18 illustrates one embodiment of the positions of the light emitting unit and the sensor unit of the clothing treatment apparatus of the present disclosure.

The light emitting unit 600 and the sensor unit 700 may be provided in different areas with respect to the bottom surface of the inner case 200. The light emitting unit 600 and the sensor unit 700 may be provided in different areas among the first area V1 and the second area V2.

The filter unit 380 may be disposed between the light emitting unit 600 and the sensor unit 700.

The light emitted from the light emitting unit 600 may penetrate the filter unit 380 and be detected by the sensor unit 700.

One of the light emitting unit 600 and the sensor unit 700 may be disposed above the filter unit 380, and the other may be disposed below the filter unit 380.

The light emitting unit 600 may be provided in the first area V1, and the sensor unit 700 may be provided in the second area V2. Alternatively, the light emitting unit 600 may be provided in the second area V2, and the sensor unit 700 may be provided in the first area V1.

That is, one of the light emitting unit 600 and the sensor unit 700 may be disposed above the body filter 386, and the other may be disposed below the body filter 386.

If the light emitting unit 600 is provided on one side of the body filter 386 and the sensor unit 700 is provided on the other side of the body filter 386, each of the light emitting unit 600 and the sensor unit 700 may be provided at the position A, B, C, or D or the position E (see FIG. 17).

For example, if the light emitting unit 600 is provided at the position E, the sensor unit 700 may be provided at at least one of the position A, B, C, or D. Conversely, if the sensor unit 700 is provided at the position E, the light emitting unit 600 may be provided at at least one of the position A, B, C, or D.

If the light emitting unit 600 or the sensor unit 700 is provided at the position E and the sensor unit 700 or the light emitting unit 600 is provided in the inner case 200, that is, at one of the positions A, B, C, and D, a reflection unit 400 configured to guide the light emitted from the light emitting unit 600 to the sensor unit 700 may be further provided. The detailed structure and position of the reflection unit 400 will be described later.

The sensor unit 700 may be provided at the position A, and the light emitting unit 600 may be provided at the position E.

The sensor unit 700 may be provided in the upper portion of the inner case 200.

For example, one of the light emitting unit 600 and the sensor unit 700 may be disposed below the filter unit 380, and the other one of the light emitting unit 600 and the sensor unit 700 may be disposed on the upper surface of the inner case 200.

Alternatively, one of the light emitting unit 600 and the sensor unit 700 may be disposed below the filter unit 380 and the hanger unit.

The sensor unit 700 may be provided at the center of the inner case 200 in the width direction.

The light emitting unit 600 may be provided below the body filter 386 or on the rear surface of the inflow body 361.

The light emitting unit 600 may be provided at the center of the machine compartment in the width direction.

The light emitting unit 600 and the sensor unit 700 may be provided on the same line in the height direction of the clothing treatment apparatus.

At this time, since the light emitted from the light emitting unit 600 should be emitted in a straight line toward the sensor unit 700, the filter cover 382 and the blocking cover 381 may not be provided.

One light emitting unit 600 and one sensor unit 700 may be provided.

Unlike the above description, the positions of the light emitting unit 600 and the sensor unit 700 may be interchanged.

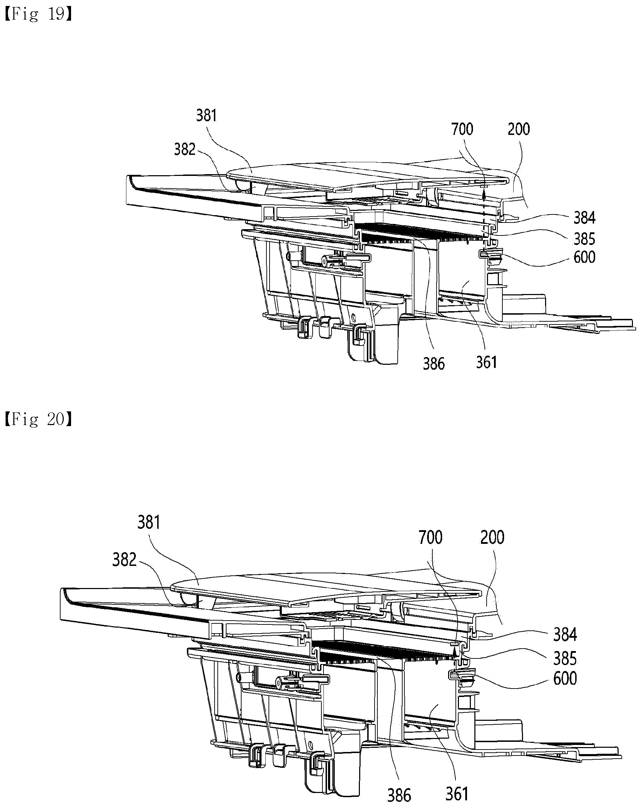

FIG. 19 illustrates another embodiment of the positions of the light emitting unit and the sensor unit of the clothing treatment apparatus of the present disclosure.

If the sensor unit 700 is provided in the upper portion of the inner case 200, the light emitted from the light emitting unit 600 may not reach the sensor unit 700 due to clothes having inside the inner case 200. As a result, the clothing treatment apparatus or the controller C may not be able to determine whether the filter is attached or detached.

Therefore, in order to minimize influence of clothes hanging in the inner case 200, the sensor unit 700 may be provided closer to the lower surface than the upper surface of the inner case 200.

In addition, the sensor unit 700 may be disposed on the inner circumferential surface of the inflow hole 231, or the like, and may be disposed below the lower surface of the inner case 200.

The light emitting unit 600 and the sensor unit 700 may be disposed below the filter cover 382.

The light emitting unit 600 and the sensor unit 700 may be disposed in the inflow body 361 to prevent exposure thereof to the inner case 200.

Referring to FIG. 17, the sensor unit 700 may be provided at the position D, and the light emitting unit 600 may be provided at the position E.

In addition, as shown in this figure, the sensor unit 700 may be provided on the lower surface of the filter cover 382.

One light emitting unit 600 and one sensor unit 700 may be provided. Accordingly, material costs and manufacturing costs of the clothing treatment apparatus may be reduced, and the clothing treatment apparatus may efficiently detect whether clothes have fallen using one sensor unit 700.

The sensor unit 700 may be disposed at a position which may identify the light emitting unit 600. For example, the sensor unit 700 may be disposed to face the light emitting unit in the height direction of the clothing treatment apparatus or in the flow path direction of the inflow duct. The sensor unit 700 may be provided on the same line as the center of the inner case in the width direction, not the center of the circulation duct in the width direction.

The sensor unit 700 may be provided at the center of the lower surface of the filter cover 382 in the width direction. Accordingly, the light emitting unit 600 and the sensor unit 700 may be provided in a straight line at the center of the inner case 200 in the width direction.

Unlike this figure, the sensor unit 700 may be provided at the exact center of the filter cover 382. Here, the sensor unit 700 is not provided on the same line as the light emitting unit 600.

The light emitting unit 600 and the sensor unit 700 may be provided on the same line in the height direction of the clothing treatment apparatus.

In addition, unlike the above description, the positions of the light emitting unit 600 and the sensor unit 700 may be interchanged.

FIG. 20 illustrates another embodiment of the positions of the light emitting unit and the sensor unit of the clothing treatment apparatus of the present disclosure.

The sensor unit 700 may be provided at the position D, and the light emitting unit 600 may be provided at the position E.

The sensor unit 700 may be provided above the body filter 386 or on the rear surface of the body cover 385.

The light emitting unit 600 may be provided below the body filter 386 or on the rear surface of the inflow body 361.

The light emitting unit 600 and the sensor unit 700 may be provided at the center of the machine compartment in the width direction.

The light emitting unit 600 and the sensor unit 700 may be provided on the same line in the height direction of the clothing treatment apparatus.

One light emitting unit 600 and one sensor unit 700 may be provided. Accordingly, material costs and manufacturing costs of the clothing treatment apparatus may be reduced, and the clothing treatment apparatus may efficiently detect whether clothes have fallen using one sensor unit 700.

Here, the filter cover and the blocking cover 381 may be omitted. Accordingly, even if the user does not install the filter cover or the blocking cover 381, it may be possible to determine whether the body filter 386 is attached or detached or the contamination level thereof.

As a result, the clothing treatment apparatus or the controller C may determine whether the body filter 386 is attached or detached or the contamination level thereof while minimizing influence of clothes provided in the inner case 200.

Unlike the above description, the positions of the light emitting unit 600 and the sensor unit 700 may be interchanged.

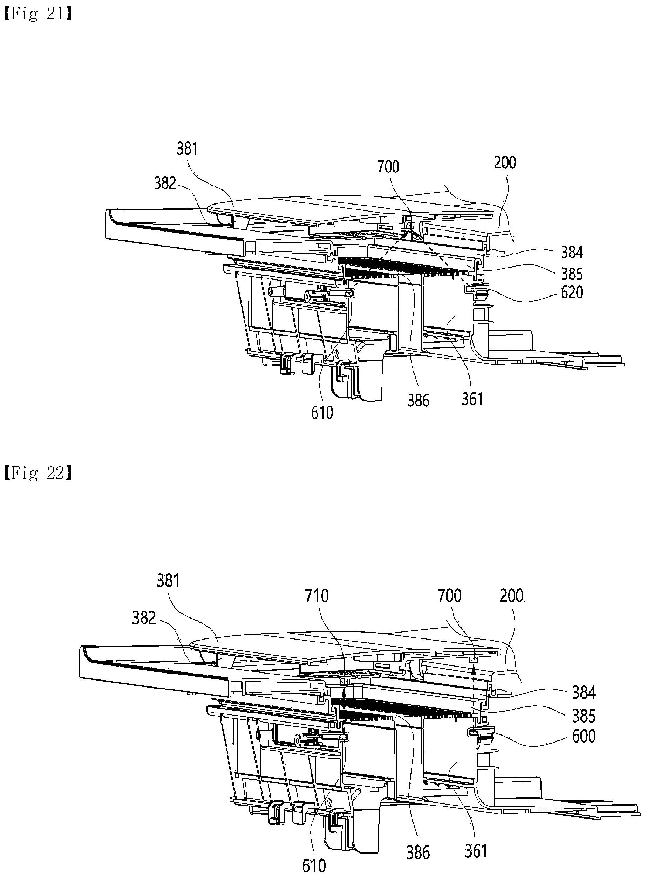

FIG. 21 illustrates another embodiment of the positions of the light emitting unit and the sensor unit of the clothing treatment apparatus of the present disclosure.

At least one sensor unit 700 and at least one light emitting unit 600 may be provided.

If two or more light emitting units 600 are provided, the amount of light received by the sensor unit 700 increases, and thus, it is possible to more accurately determine whether the filter is attached or detached.

The number of the light emitting units 600 and the number of the sensor units 700 may not be the same, and if two or more light emitting units 600 are provided, two or more sensor unis 700 may also be provided.

Even if two or more light emitting units 600 are provided, only one sensor unit 700 may be provided.

The light emitting units 600 may further include a first light emitting unit 610 and a second light emitting unit 620 provided to emit light toward the body filter 386.

The first light emitting unit 610 and the second light emitting unit 620 may be provided at the position E.

The first light emitting unit 610 and the second light emitting unit 620 may be provided on one surface of the inflow body 361. The first light emitting unit 610 and the second light emitting unit 620 may be disposed on the inner surface of the inflow body 361 to be exposed.

For example, the first light emitting unit 610 may be provided on the front surface of the inflow body 361, and the second light emitting unit 620 may be provided on the rear surface of the inflow body 361.

The first light emitting unit 610 and the second light emitting unit 620 may be provided to face each other in the forward and rearward directions of the inflow body 361.

The first light emitting unit 610 may be provided so that light emitted from the first light emitting unit 610 passes through the body filter 386 and reaches the sensor unit 700. The second light emitting unit 620 may be provided so that light emitted from the second light emitting unit 620 passes through the body filter 386 and reaches the sensor unit 700.

The sensor unit 700 may be provided at the position D.

The sensor unit 700 may be provided on the lower surface of the filter cover 382.

The sensor unit 700 may be provided at the center of the lower surface of the filter cover 382 in the width direction.

The sensor unit 700 may be provided at the exact center of the filter cover 382. Thereby, it may receive evenly the light emitted from the first light emitting unit 610 and the second light emitting unit 620.

As a result, since the light emitted from the light emitting units 600 evenly penetrates the body filter 386, the clothing treatment apparatus or the controller C may more accurately measure the contamination level of the body filter 386.

In addition, since the sensor unit 700 receives the light emitted from the light emitting units 600 all at once, the clothing treatment apparatus or the controller C may easily control the sensor unit 700 at once.

FIG. 22 illustrates another embodiment of the positions of the light emitting unit and the sensor unit of the clothing treatment apparatus of the present disclosure.

The light emitting units 600 and the sensor units 700 may be provided in the same number. If a plurality of light emitting units 600 is provided, a plurality of sensor units 700 may be provided.

For example, if two light emitting units 600 are provided, two sensor units 700 may be provided.

The light emitting units 600 may further include a first light emitting unit 610 and a second light emitting unit 620 provided to emit light toward the body filter 386.

The first light emitting unit 610 and the second light emitting unit 620 may be provided at the position E.

The first light emitting unit 610 and the second light emitting unit 620 may be provided on one surface of the inflow body 361. For example, the first light emitting unit 610 may be provided on the front surface of the inflow body 361, and the second light emitting unit 620 may be provided on the rear surface of the inflow body 361.

The first light emitting unit 610 and the second light emitting unit 620 may be provided in parallel in the forward and rearward directions of the inflow body 361.

The first light emitting unit 610 may be provided so that light emitted from the first light emitting unit 610 passes through the body filter 386 and reaches the sensor unit 700. The second light emitting unit 620 may be provided so that light emitted from the second light emitting unit 620 passes through the body filter 386 and reaches the sensor unit 700.

The sensor units 700 may be provided to face at least one light emitting unit 600.

The sensor units 700 may further include a first sensor unit 710 disposed above the first light emitting unit 610 to receive the light emitted from the first light emitting unit 610, and a second sensor unit 720 disposed above the second light emitting unit 620 to receive the light emitted from the second light emitting unit 620.

The first sensor unit 710 and the second sensor unit 720 may be provided at the position D.

The first sensor unit 710 may be disposed to face the first light emitting unit 610, and the second sensor unit 720 may be disposed to face the second light emitting unit 620.

The first sensor unit 710 and the second sensor unit 720 may be provided on the lower surface of the filter cover 382.

The first sensor unit 710 and the second sensor unit 720 may be provided at the center of the lower surface of the filter cover 382 in the width direction or the center of the inner case 200 in the width direction.

The first sensor unit 710 and the second sensor unit 720 may be provided in parallel in the forward and rearward directions of the filter cover 382.

As a result, since the light emitted from the light emitting units 600 evenly penetrates the body filter 386, the clothing treatment apparatus or the controller C may more accurately measure not only whether the body filter 386 is attached or detached but also the contamination level of the body filter 386.

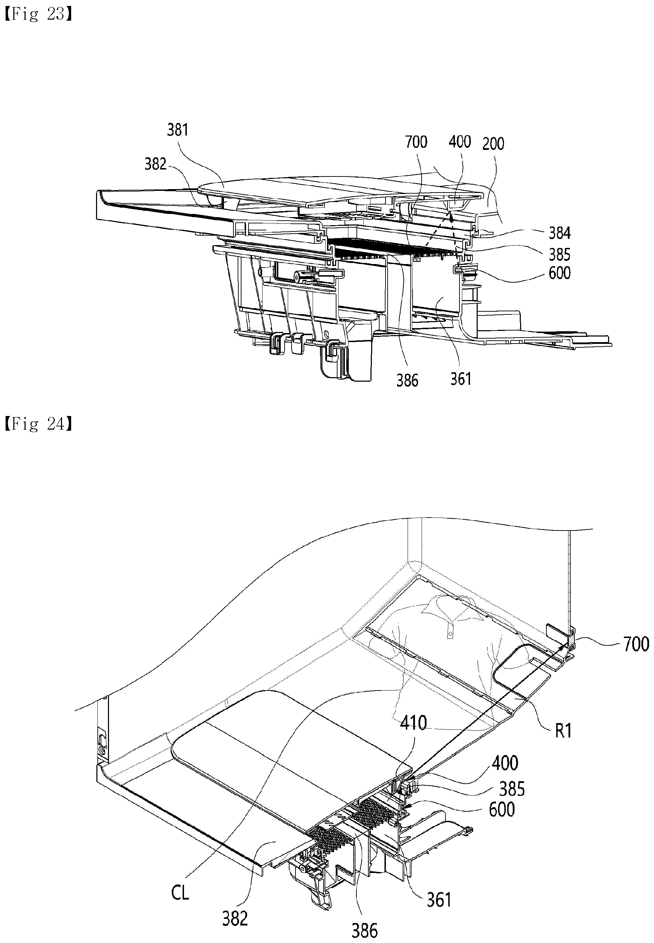

FIG. 23 illustrates another embodiment of the positions of the light emitting unit and the sensor unit of the clothing treatment apparatus of the present disclosure.

The light emitting unit 600 and the sensor unit 700 may be disposed below the body filter 386. That is, the light emitting unit 600 and the sensor unit 700 may be provided at the position E.

One light emitting unit 600 and one sensor unit 700 may be provided. Accordingly, material costs and manufacturing costs of the clothing treatment apparatus may be reduced, and the clothing treatment apparatus may efficiently detect whether clothes have fallen using one sensor unit 700.

The sensor unit 700 may be provided below the body filter 386.

The sensor unit 700 may be provided below the body cover 385.

The sensor unit 700 may be provided below the perforated plate of the body cover 385.

The sensor unit 700 may be provided on one surface of the inflow body 361 extending from at least one of the first rib or the second rib.

The sensor unit 700 may be provided to be in contact with the lower surface of the perforated plate of the body cover 385 and one surface of the inflow body 361 extending from at least one of the first rib or the second rib.

Unlike the above figure, the sensor unit 700 may be provided on one surface of the inflow body 361, and the light emitting unit 600 may be provided on one surface of the body cover 385.

The clothing treatment apparatus may further include the reflection unit 400 provided to guide the light emitted from the light emitting unit 600 to the sensor unit 700 through the body filter 386.

The reflection unit 400 may be provided below the filter cover 382.

The refection unit 400 may be provided in a straight line with the light emitting unit 600.

The reflection unit 400 may be provided so that the light emitted from the light emitting unit 600 reaches the sensor unit 700. The detailed structure and position of the reflection unit 400 will be described later.

Therefore, if the body filter 386 is not installed, the light emitted from the light emitting unit 600 penetrates the body cover 385 and is reflected by the reflection unit 40. The light reflected by the reflection unit 400 reaches the sensor unit 700, and the sensor unit 700 may detect an increase in illuminance.

FIG. 24 illustrates another embodiment of the positions of the light emitting unit and the sensor unit of the clothing treatment apparatus of the present disclosure.

The light emitting unit 600 and the sensor unit 700 may be installed at any positions as long as the sensor unit 700 detects the light emitted from the light emitting unit 600.

As shown in this figure, the light emitting unit 600 may be provided at the position E, and the sensor unit 700 may be provided at the position C.

The sensor unit 700 may be disposed closer to the filter unit 380 than the hanger unit.

The light emitting unit 600 and the sensor unit 700 may be provided with the body filter 386 interposed therebetween. Accordingly, the light emitting unit 600 may be provided so that the light emitted from the light emitting unit 600 penetrates the body filter 386 and is detected by the sensor unit 700.

The light emitting unit 600 may be provided below the body filter 386. Accordingly, deterioration of the performance of the light emitting unit 600 due to dust, etc. filtered out by the body filter 386 may be prevented, and exposure of the user's eyes to light may be prevented.

Further, power supply and repair may be facilitated, and influence of clothes hanging in the inner case 200 may be minimized.

The light emitting unit 600 may be disposed in the inflow body 361, and the sensor unit 700 may be provided in the inner case 200 or the door 40.

The light emitting unit 600 may be provided on the rear surface of the inflow body 361 to emit light to the upper portion of the inflow body 361 among the position E. That is, the light emitting unit 600 may be provided to emit light to the upper portion of the first inlet or the third inlet. The shielding member configured to shield the second inlet may be further coupled to the second inlet of the inflow body 361. Therefore, the light emitting unit 600 may be provided so that the light may not be emitted to the upper portion of the second inlet, and may thus be preferably provided on the front or rear surface rather than the side surface of the inflow body 361.

The light emitting unit 600 may further include a light installation unit in which a light source may be installed. The light installation unit may be provided by penetrating the front or rear surface of the inflow body 361.

Since the light emitting unit 600 is provided below the body filter 386, the clothing treatment apparatus or the controller C may determine not only whether the body filter 386 is attached or detached but also the contamination level of the body filter 386 using the fact that the sensor unit 700 detects a change in the illuminance of the light emitted from the light emitting unit 600.

Meanwhile, the machine compartment may be provided to be located in the left side of the clothing treatment apparatus. The center of the machine compartment in the width direction and the center of the clothing treatment apparatus in the width direction may not be the same. The center of the clothing treatment apparatus in the width direction may be provided on the right side of the machine compartment.

Therefore, the light emitting unit 600 may be provided on the same line as the center of the clothing treatment apparatus in the width direction not the center of the machine compartment in the width direction.

One light emitting unit 600 and one sensor unit 700 may be provided. Accordingly, material costs and manufacturing costs of the clothing treatment apparatus may be reduced, and the clothing treatment apparatus may efficiently detect whether clothes have fallen using one sensor unit 700.

The inner case 200 has a longer length from the machine compartment to the rear surface of the inner case 200 than the length from the machine compartment to the door 40. Therefore, the light emitting unit 600 is preferably provided so that the light emitted therefrom is directed to the rear portion of the inner case 200. Accordingly, the sensor unit 700 may be provided on the rear surface of the inner case 200 to face the inside of the inner case 200 among the position D.

The sensor unit 700 may be provided at the center of the rear surface of the inner case 200 in the width direction. Accordingly, the sensor unit 700 and the light emitting unit 600 may be provided in a straight line at the center of the inner case 200 in the width direction.

Contrary to the above description, the light emitting unit 600 may be provided on the rear surface of the inner case 200, and the sensor unit 700 may be provided on the rear surface of the inflow body 361.

In order for the clothing treatment apparatus or the controller C to determine whether the body filter 386 is attached or detached or the contamination level of the body filter 386, the light emitted from the light emitting unit 600 should penetrate the body filter 386, and thus the light emitting unit 600 is preferably provided below the body filter 386.

However, if the light emitting unit 600 is provided below the body filter 386, the light emitted from the light emitting unit 600 may not reach the sensor unit 700 provided on the rear surface of the inner case 200 due to the angle of the light and a step between the body cover 385 and the inner case 200.

In order for the sensor unit 700 to detect a change in the illuminance of the light emitted from the light emitting unit 600, the light emitted from the light emitting unit 600 should be guided toward the lower portion of the inner case 200 and ultimately reach the sensor unit 700.

Therefore, in order to guide the light emitted from the light emitting unit 600 to the sensor unit 700 and secure the amount of illuminance required for detection, the reflection unit 400 that is disposed above the light emitting unit 600 and the filter unit 380 to guide the light emitted from the light emitting unit 600 to the sensor unit 700 may be further provided.

The reflection unit 400 may be provided in a number and at a position and angle that enable the reflection unit 400 to reflect the light emitted from the light emitting unit 600 so that the sensor unit 700 may detect the reflected light.

The reflection unit 400 may be provided to reflect the light emitted from the light emitting unit 600 to guide the reflected light to the sensor unit 700, regardless of where the light emitting unit 600 and the sensor unit 700 are provided.

The reflection unit 400 is provided above the light emitting unit 600 to be disposed in a straight line with the light emitting unit 600. The reflection unit 400 may be provided so that the light emitted from the light emitting unit 600 directly reaches the reflection unit 400.

The reflection unit 400 may be provided above the body filter 386. The reflection unit 400 may be provided on one surface of the body cover 385.

The reflection unit 400 may be disposed between the filter cover 382 and the body filter 386.

The reflection unit 400 may be provided on the lower surface of the filter cover 382. In order to overcome the step between the body cover 385 and the bottom surface of the inner case 200 and allow the reflection unit 400 to guide the light emitted from the light emitting unit 600 to the sensor unit 700 provided on the rear surface of the inner case 200, the reflection unit 400 is preferably provided on the lower surface of the filter cover 382.

If the reflection unit 400 is provided on the lower surface of the filter cover 382, the filter cover 382 may further include a reflection unit 400 installation unit for installing the reflection unit 400 on the lower surface of the filter cover 382. The detailed structure of the filter cover 382 will be described later.

One light emitting unit 600 and one sensor unit 700 may be provided.

The light emitting unit 600 may be provided on the rear surface of the inflow body 361 to be located at the center of the clothing treatment apparatus in the width direction.

The sensor unit 700 may be provided at the center of the rear surface of the inner case 200 in the width direction.

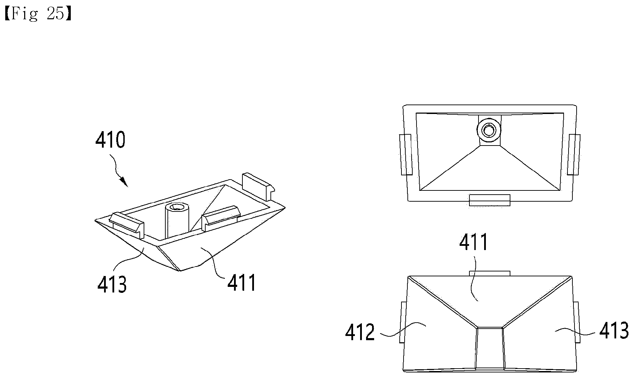

FIG. 25 illustrates the structure of a first reflector.

FIG. 25(a) is a perspective view of the first reflector 410, FIG. 25(b) illustrates the inside of the second reflector 410, and FIG. 25(c) illustrates the outside of the first reflector 410.

The reflection unit 400 may be provided to reflect the light from the light emitting unit 600.

The reflection unit 400 may include the first reflector 410 that reflects the light from the light emitting unit 600.

The first reflector 410 may be disposed at a height corresponding to the light emitting unit 600.

The first reflector 410 may be provided on the lower surface of the filter cover 382.

The first reflector 410 may be provided at the center of the filter cover 382 in the width direction.

As shown in this figure, the first reflector 410 may be provided with a first reflective surface 411 that directly reflects the light emitted from the light emitting unit 600 to the sensor unit 700.

The first reflective surface 411 may be provided to face the rear portion of the clothing treatment apparatus.

The first reflector 410 may be provided with hooks 414 that may be coupled to the filter cover 382.

The clothing treatment apparatus may further include a first route R1 in which the light emitted from the light emitting unit 600 is guided to the sensor unit 700 via the first reflective surface 411.

The first route R1 may pass through the lower portion of the inner case 200. The first route R1 may be close to the bottom surface of the inner case 200.

If the body filter 366 is not installed, the light emitted from the light emitting unit 600 reaches the sensor unit 700 along the first route R1. Therefore, the sensor unit 700 may detect an abnormal increase in illuminance, and the clothing treatment apparatus or the controller C may determine whether the filter is attached or detached.

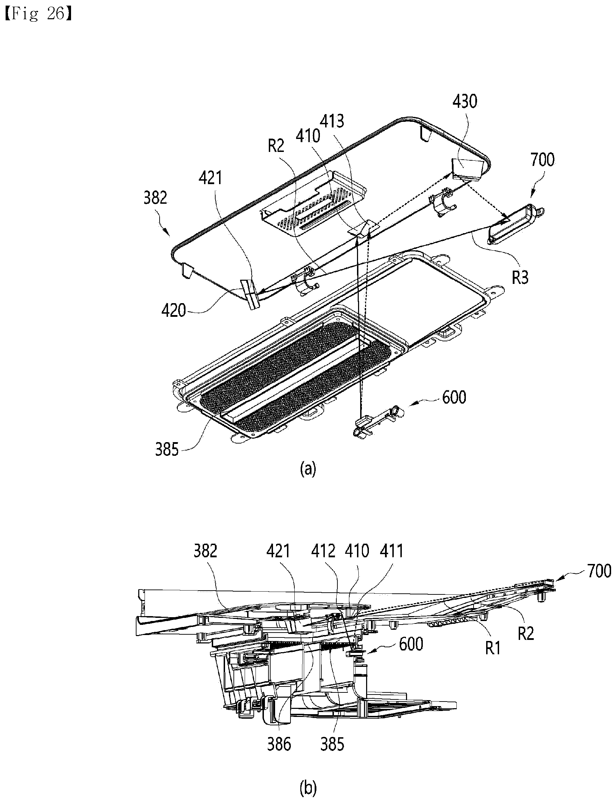

FIG. 26 illustrates one embodiment of the reflection unit of the clothing treatment apparatus of the present disclosure.

FIG. 26(a) is an exploded perspective view of the filter unit 380, and FIG. 26(b) is a cross-sectional view of the machine compartment cut in the horizontal direction.

The first reflector 410 alone may not sufficiently transmit the light emitted from the light emitting unit 400 to the sensor unit 700.

Accordingly, as shown in FIG. 26(a), the reflection unit 400 may further include a second reflector 420 and a third reflector 430 disposed symmetrically on both sides of the first reflector 410 in order to sufficiently transmit the light emitted from the light emitting unit 600 to the sensor unit 700.

The second reflector 420 and the third reflector 430 may be provided on the lower surface of the filter cover 382 close to both side surfaces of the filter cover 382.

Here, the first reflector 410 may further include a second reflective surface 412 that reflects the light from the light emitting unit 600 to the second reflector 420, and a third reflective surface 413 that reflects the light from the light emitting unit 600 to the third reflector 430.

In addition, the second reflector 420 may have a fourth reflective surface 421 that reflects the light reflected by the second reflective surface 412 toward the sensor unit 700. The third reflector 430 may have a fifth reflective surface 431 that reflects the light reflected by the third reflective surface 413 toward the sensor unit 700. Thus, the light reflected by the fourth and fifth reflective surfaces 421 and 431 may be detected by the sensor unit 700.

That is, the first reflector 410 may reflect the light emitted from the light emitting unit 600 toward the second reflector 420 and the third reflector 430. The light reflected by the second reflector 420 and the third reflector 430 may be detected by the sensor unit 700.

The clothing treatment apparatus may further include a second route R2 in which the light emitted from the light emitting unit 600 is guided to the sensor unit 700 via the second reflective surface 412 of the first reflector 410 and the fourth reflective surface 421 of the second reflector 420.

The clothing treatment apparatus may further include a third route R3 in which the light emitted from the light emitting unit 600 is guided to the sensor unit 700 via the third reflective surface 413 of the first reflector 410 and the fifth reflective surface 431 of the third reflector 430.

Referring to FIG. 26(b), if the filter is not installed, the light emitted from the light emitting unit 600 reaches the sensor unit 700 along the second route R2 and the third route R3. Therefore, the sensor unit 700 may detect an abnormal increase in illuminance, and the clothing treatment apparatus or the controller C may determine whether the filter is attached or detached.

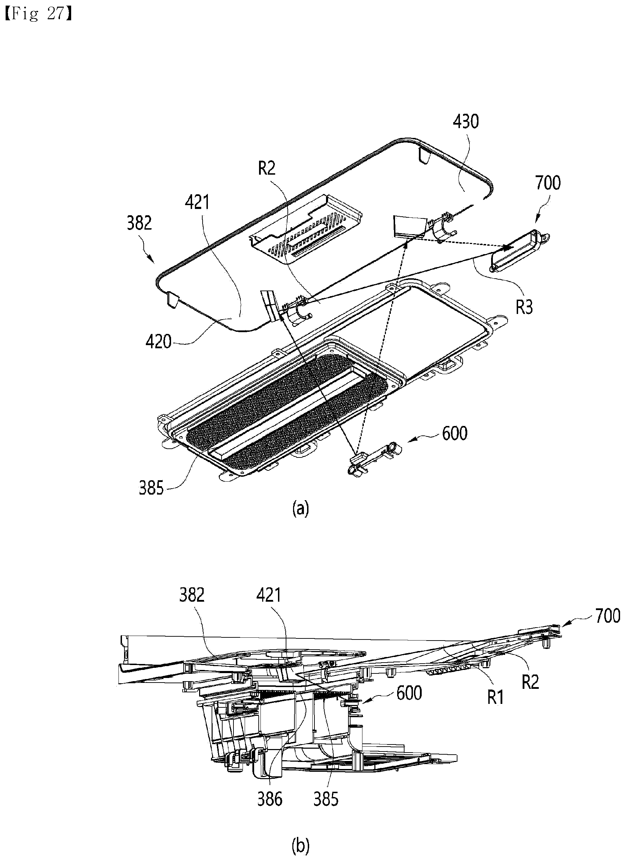

FIG. 27 illustrates another embodiment of the reflection unit of the clothing treatment apparatus of the present disclosure.

If the reflection unit 400 includes the first reflector 410, the second reflector 420, and the third reflector 430, as shown in FIG. 26, when the clothes have fallen to block at least one of the first route R1, the second route R2, or the third route R3, the sensor unit 700 may detect about 0-66% of the light emitted from the light emitting unit 600. However, when the body filter 386 is installed and the clothes do not fall, the sensor unit 700 may detect about 60-70% of the light emitted from the light emitting unit 600. Therefore, there is an overlapping range of the amounts of light detected by the sensor unit 700 when the clothes have fallen and when the clothes do not fall, and if about 66% or less of the light is detected, it is not possible to detect whether clothes have fallen, and thus there is a possibility that the accuracy of determining whether the clothes have fallen will decrease.

Therefore, in order to eliminate the overlapping range, the clothing treatment apparatus may be provided such that, if the light emitting unit 600 is provided below the body filter 386 and the sensor unit 700 is provided in the inner case 200 or the door 40 to be disposed closer to the filter unit 380 than the hanger unit 510, the reflection unit 400 includes only the second reflector 420 and the third reflector 430 without the first reflector 410.

The second reflector 420 and the third reflector 430 are provided so that the light emitted from the light emitting unit 600 may pass through the second route R2 and the third route R3 through the body filter 386.

If the clothes have fallen onto the bottom surface of the inner case 200, the clothes may block at least one of the second route R2 or the third route R3.

Therefore, the controller C may determine whether the clothes have fallen by detecting a change in the illuminance of the light emitted from the light emitting unit 600 by the sensor unit 700.

Specifically, when the body filter 386 is installed and the clothes do not fall, the sensor unit 700 may detect about 60-70% of the light emitted from the light emitting unit 600. At this time, if the clothes have fallen and block at least one of the second route R2 or the third route R3, the sensor unit 700 may detect about 0-50% of the light.

Thereby, there is no overlapping range of the amounts of light detected by the sensor unit 700 when the clothes have fallen and when the clothes do not fall, and thus the controller C may accurately determine whether the clothes have fallen and whether the body filter 36 is attached or detached with only the second reflector 420 and the third reflector 430 disposed on both sides of the filter cover 382.

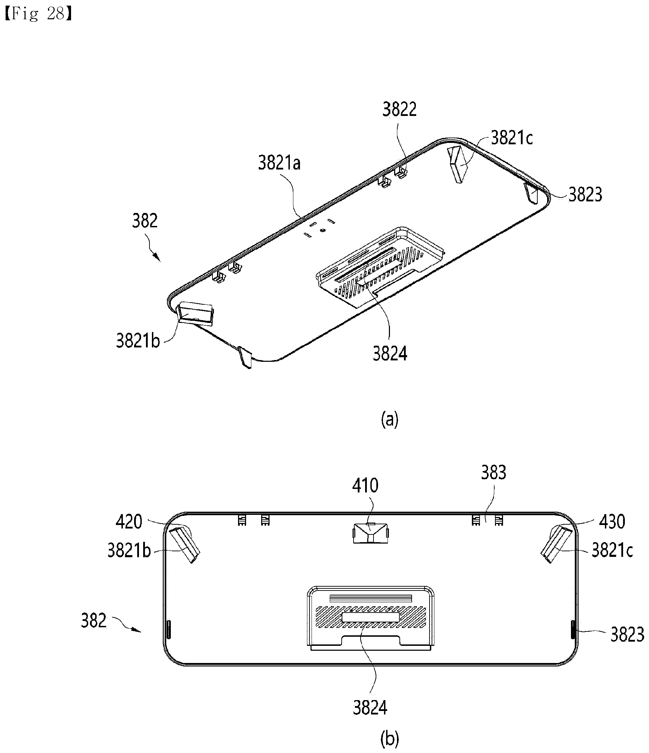

FIG. 28 illustrates the structure of the filter cover of the clothing treatment apparatus of the present disclosure.

The lower perspective view of the filter cover 382 and the bottom view of the filter cover 382 provided with the reflection units 400 are illustrated.

The filter cover 382 may have a first reflector installation unit 3821a configured to install the first reflector 410 on the lower surface of the filter cover 382.

The filter cover 382 may further include a second reflector installation unit 3821b configured to install the second reflector 420 on the lower surface of the filter cover 382, and a third reflector installation unit 3821c configured to install the third reflector 430 on the lower surface of the filter cover 382. The second reflector installation unit 3821b and the third reflector installation unit 3821c may be provided symmetrically on both sides of the first reflector installation unit 3821a.

The filter cover 382 may have link installation units 3822 configured to install links 383 detachably coupled to the filter fixing unit 384 on the lower surface of the filter cover 382.

The filter cover 382 may have hooks 3823 supported by the filter fixing unit 384 on the lower surface of the filter cover 382.

The filter cover 382 may have an aroma sheet installation unit 3824 configured to install an aroma sheet capable of deodorizing air on the lower surface of the filter cover 382.

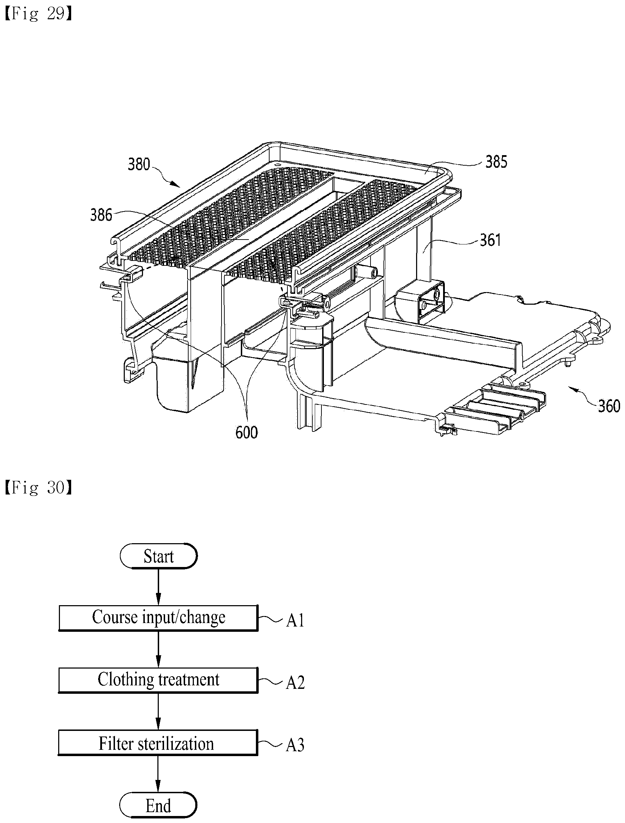

FIG. 29 illustrates one embodiment of the positions of the light emitting unit and the sensor unit for sterilizing the filter unit of the clothing treatment apparatus of the present disclosure.

Meanwhile, the conventional clothing treatment apparatus had a problem in that dust due to clothing treatment could accumulate on the body filter 386 and cause microorganisms harmful to the human body to occur in the body filter 386. Since the user had to directly remove the body filter 386 having the accumulated dust after the cycle was completed, there was a problem of poor convenience and hygiene.

The conventional clothing treatment apparatus had a sterilization effect by steam in a steam cycle during the clothing treatment process, but there was a problem in that microorganisms could remain in the body filter 386 even after the end of the cycle.

In addition, as user's interest in hygiene of clothing treatment apparatuses increases, there is a need to enhance hygiene of not only clothes but also the clothing treatment apparatuses that treat clothes.

Accordingly, the present disclosure provides the clothing treatment apparatus including the light emitting unit 600 that performs a function of sterilizing the body filter 386 using light.

The light emitting unit 600 may be provided to sterilize the body filter 386 through the light.

Since the body filter 386 is located in the upper portion of the machine compartment and is vulnerable to heat or temperature, there is an advantage in that the inside of the machine compartment is not affected if the body filter 386 is sterilized using light.

The clothing treatment apparatus may include the light emitting unit 600 that emits light of a specific wavelength toward the body filter 386.

The light emitting unit 600 may be provided at any position as long as it is provided to evenly radiate light throughout the entire area of the body filter 386.

The filter unit 380 may further include the reflection unit 400 to evenly radiate light throughout the entire area of the boy filter 386.

The light emitting unit 600 may be provided to emit light in the ultraviolet spectrum.

The light emitting unit 600 may be provided to emit visible light having a wavelength of 405 nm. Visible light having the wavelength of 405 nm is harmless to the human body, and microorganisms may be sterilized with the visible light alone.

A photocatalyst may be further added to the body filter 386. A chemical substance that emits a sterilizing substance upon coming into contact with visible light may be applied to the filter unit 380.

FIG. 30 illustrates a control method of sterilizing the filter unit of the clothing treatment apparatus of the present disclosure.

The clothing treatment apparatus may further include a filter sterilization step A3 of sterilizing the body filter 386 using the light emitting unit 600.

Specifically, if the user turns on the clothing treatment apparatus and power is supplied thereto, a course input/change step A1 may be performed.

The clothing treatment apparatus may further include an input unit I provided on the door 40 or the cabinet 100 to receive an input or change command of a course.

**In** the course input/change step, the user may select and input a desired course through the input unit I. When the user selects the desired course through the input unit I, the compressor, the heater unit 840, the vibration unit 520, etc. start to operate accordingly. For example, if the general clothing treatment course is selected, the heater unit 840 starts to operate to generate steam.

After the course input/change step A1, a clothing treatment step A2 may be performed.

The clothing treatment step A2 may include at least one of a steam injection step of supplying steam to the inside of the inner case 200 by operating the heater unit 840 or a drying step of supplying hot air to the inside of the inner case 200 by operating the compressor 342.

As shown in this figure, the filter sterilization step A3 may be automatically performed after the clothing treatment step A2 has been completed. In the filter sterilization step A3, the filter may be sterilized using light emitted from the light emitting unit 600 regardless of the clothing treatment step, such as steam injection or drying.

Therefore, the body filter 386 may be automatically sterilized without the user having to worry about sterilizing the body filter 386, thereby providing convenience.

The user may also turn on/off the function of automatically performing the filter sterilization step A3.

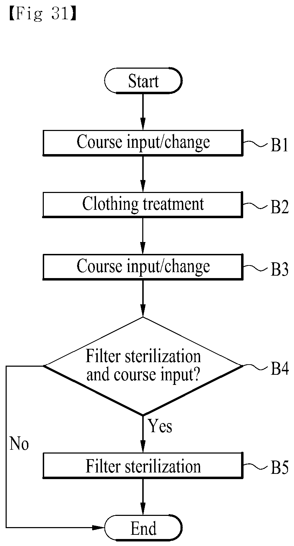

FIG. 31 illustrates another control method of sterilizing the filter unit of the clothing treatment apparatus of the present disclosure.

This figure shows that a filter sterilization input step B4 is performed if the function of automatically performing the filter sterilization is turned off.

The filter sterilization input step B4 may be performed by user's setting in a sterilization course input step B3 after a clothing treatment step B2 has been completed. Accordingly, the body filter 386 may be sterilized only if the user wants. Specifically, a filter sterilization step B5 may be performed only if a command to perform the filter sterilization step B5 is input in the sterilization course input step B3.

If the filter sterilization step B5 is performed, a step using at least one of the heat pump systems (the compressor, the steam generator, or the like) may not be performed.

Regardless of whether the function of automatically performing the filter sterilization is turned on/off, the filter sterilization step may be performed whenever the filter sterilization course is input in the course input step B3 during standby (when storing clothes).

Therefore, the clothing treatment apparatus of the present disclosure may eliminate harmful bacteria, viruses, etc. and prevent reproduction thereof by sterilizing the filter using the light emitting unit 600.

Although the present disclosure has been illustrated and described in relation to specific embodiments, it will be apparent to those skilled in the art that the present disclosure may be variously improved and changed without departing from the technical idea of the present disclosure provided by the following claims.

## Claims

1. A clothing treatment apparatus comprising:
a cabinet having an opening formed in a front portion thereof;
an inner case provided in the cabinet to provide a space configured to accommodate clothes;
a door coupled to the cabinet to open and close the opening;
a machine compartment provided below a bottom surface of the inner case and comprising an inflow body configured to allow air to be introduced thereinto from the inner case and a discharge duct configured to discharge air to the inner case;
a filter unit mounted on the inflow body to filter out dust introduced into the inflow body;
a light emitting unit disposed to be spaced apart from the filter unit and provided to emit light toward the filter unit; and
a sensor unit provided to detect the light emitted from the light emitting unit and having passed through the filter unit; and
further comprising a controller provided to detect at least one of whether the filter unit is configured to be attached or detached or a contamination level of the filter unit depending on an amount of the light emitted from the light emitting unit, detected by the sensor unit.

2. The clothing treatment apparatus according to claim 1, wherein the filter unit is configured to be disposed between the light emitting unit and the sensor unit.

3. The clothing treatment apparatus according to claim 2, wherein one of the light emitting unit and the sensor unit is configured to be disposed above the filter unit, and a remaining one is disposed below the filter unit.

4. The clothing treatment apparatus according to claim 3, wherein:
the light emitting unit is configured to be disposed in the inflow body; and
the sensor unit is configured to be disposed in the inner case or the door.

5. The clothing treatment apparatus according to claim 4, wherein the sensor unit is disposed closer to the filter unit than a hanger unit.

6. The clothing treatment apparatus according to claim 4, further comprising a reflection unit is configured to dispose above the light emitting unit and the sensor unit and provided to guide the light emitted from the light emitting unit to the sensor unit.

7. The clothing treatment apparatus according to claim 6, wherein the filter unit comprises:
a body filter mounted on the inflow body; and
a filter cover disposed above the body filter,
wherein the reflection unit is configured to be disposed between the filter cover and the body filter.

8. The clothing treatment apparatus according to claim 3, wherein:
one of the light emitting unit and the sensor unit is disposed below the filter unit; and
a remaining one of the light emitting unit and the sensor unit is configured to be disposed on an upper surface of the inner case.

9. The clothing treatment apparatus according to claim 3, further including a hanger unit provided in an upper portion of the inner case to hold the clothes, and one of the light emitting unit and the sensor unit may be disposed below the filter unit and the hanger unit.

10. The clothing treatment apparatus according to claim 1, wherein the light emitting unit and the sensor unit are configured to be disposed in the inflow body to prevent exposure thereof to the inner case.

11. The clothing treatment apparatus according to claim 10, wherein the filter unit comprises:
a body filter mounted on the inflow body; and
a filter cover disposed above the body filter,
wherein the light emitting unit and the sensor unit are configured to be disposed below the filter cover.

12. The clothing treatment apparatus according to claim 11, wherein the light emitting unit and the sensor unit are disposed below the body filter, and wherein the clothing treatment apparatus further includes a reflection unit provided to guide the light emitted from the light emitting unit to the sensor unit through the body filter.

13. A clothing treatment apparatus comprising:
a cabinet;
an inner case provided in the cabinet to provide a space configured to accommodate clothes;
a machine compartment provided below the inner case and comprising an inflow body configured to allow air to be introduced thereinto from the inner case and a discharge duct configured to discharge air to the inner case;
a filter unit mounted on the inflow body to filter out dust introduced into the inflow body;
a light emitting unit disposed to be spaced apart from the filter unit and provided to emit light toward the filter unit;
a sensor unit provided to detect the light emitted from the light emitting unit and having passed through the filter unit; and
a controller provided to detect whether the filter unit is attached or detached depending on an amount of the light emitted from the light emitting unit, detected by the sensor unit,
wherein:
the sensor unit is configured to detect a first value when the filter unit is installed on the inflow body; and
the controller determines that the filter unit is not configured to be installed on the inflow body when the sensor unit detects a value greater than the first value.

14. The clothing treatment apparatus according to claim 13, wherein:
the machine compartment further comprises a steam generator configured to generate steam to be supplied to the inner case, and a compressor configured to compress a refrigerant provided to exchange heat with air supplied to the inner case; and
the controller is provided to shut down operation of at least one of the steam generator or the compressor upon determining that the filter unit is not installed.

15. The clothing treatment apparatus according to claim 13, wherein:
the sensor unit detects a second value when the filter unit is not contaminated; and
the second value is smaller than the first value, and the controller determines that the filter unit is contaminated when a value smaller than the second value is detected.

16. The clothing treatment apparatus according to claim 15, further comprising a display provided on one of the cabinet and the door to display a state of the filter unit,
wherein the controller is provided to display contamination information of the filter unit on the display upon determining that the filter unit is contaminated.

17. A clothing treatment apparatus comprising:
a cabinet;
an inner case provided in the cabinet to provide a space configured to accommodate clothes;
a machine compartment provided below the inner case and comprising an inflow body configured to allow air to be introduced thereinto from the inner case and a discharge duct configured to discharge air to the inner case;
a filter unit mounted on the inflow body to filter out dust introduced into the inflow body; and
a light emitting unit disposed to be spaced apart from the filter unit and provided to emit light toward the filter unit,
wherein the light emitting unit is provided to sterilize the body filter through the light.

18. The clothing treatment apparatus according to claim 17, wherein the light emitting unit is provided to emit visible light having a wavelength of 405 nm.

19. The clothing treatment apparatus according to claim 18, wherein a chemical substance configured to emit a sterilizing substance upon coming into contact with the visible light is applied to the filter unit.

20. The clothing treatment apparatus according to claim 17, wherein the light emitting unit is configured to emit light in an ultraviolet spectrum.
